# FASCICULE DE BREVET EUROPEEN

(11) **EP 1 133 491 B1**
(45) Date de publication et mention de la délivrance du brevet: **19.02.2003**
(21) Numéro de dépôt: 99956121.0
(22) Date de dépôt: 23.11.1999
(51) Int. Cl.: C07D 405/12, A61K 31/495

(54) **DERIVES DU BENZOFURANE, LEUR PROCEDE DE PREPARATION, LEUR APPLICATION COMME MEDICAMENTS ET LES COMPOSITIONS PHARMACEUTIQUES LES RENFERMANT**
BENZOFURANDERIVATE, VERFAHREN ZU IHRER HERSTELLUNG, IHRE ANWENDUNG ALS MEDIKAMENT UND SIE ENTHALTENDE PHARMAZEUTISCHE ZUBEREITUNGEN
NOVEL BENZOFURANE DERIVATIVES, PREPARATION METHOD, USE AS MEDICINES AND PHARMACEUTICAL COMPOSITIONS CONTAINING SAME

(30) Priorité: 24.11.1998 FR 9814779
(43) Date de publication de la demande: 19.09.2001
(73) Titulaire: Aventis Pharma S.A., 92160 Antony (FR); Genentech, Inc., South San Francisco, CA 94080-4990 (US)
(72) Inventeur: CARNIATO, Denis, F-06800 Cagnes sur Mer (FR); GADEK, Thomas, R., Oakland, CA 94611 (US); GOURVEST, Jean-François, F-77410 Claye-Souilly (FR); KNOLLE, Jochen, D-65830 Kriftel (DE); PEYMAN, Anurschirwan, D-65779 Kelkheim (DE); BODARY, Sarah, C., San Bruno, CA 94066 (US)
(74) Mandataire: Rousseau, Pierrick Edouard
(86) Numéro de dépôt international: FR9902879
(87) Numéro de publication internationale: WO00031070

(56) Documents cités:
- EP-A- 0 820 991
- WO-A-94/08962
- WO-A-95/32710

## Description

La présente invention a pour objet de nouveaux dérivés du benzofurane, leur procédé de préparation, leur application comme médicaments et les compositions pharmaceutiques les renfermant.

L'invention a pour objet les composés de formule (I) 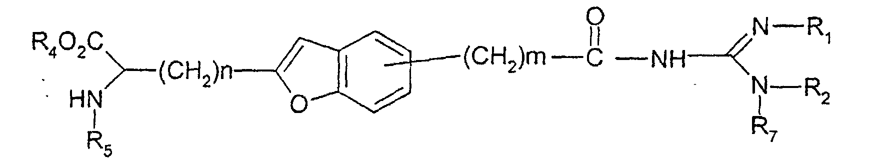 dans laquelle R₁, R₂, R₄, R₅ et R₇ ont les significations indiquées plus bas, leurs sels physiologiquement acceptables et leurs prodrugs. Les composés de formule (I) sont des composés ayant une activité pharmacologique et sont donc utilisables à titre de médicaments. Ce sont des antagonistes du récepteur de la vitronectine et des inhibiteurs de l'adhésion cellulaire et ils inhibent la résorption osseuse médiée par les ostéoclastes. Ils sont donc utiles pour le traitement thérapeutique ou la prophylactique de maladies qui sont dues, au moins en partie, à une augmentation non désirée de la résorption osseuse, par exemple l'ostéoporose. L'invention de plus a pour objet les procédés de préparation des composés de formule (I), leur utilisation, en particulier à titre ue médicament et les compositions pharmaceutiques les renfermant. L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. La majorité des désordres au niveau de l'os sont basés sur un équilibre perturbé entre la formation osseuse et la résorption osseuse. L'ostéoporose est caractérisée par une perte sèche de cette matrice osseuse. Un ostéoclaste mature activé résorbe l'os, après adhésion à la matrice osseuse, via la sécrétion d'enzymes protéolytiques et de protons à l'intérieur de la zone d'adhésion, aboutissant à des dépressions ou des creusements de la surface de l'os qui apparaissent au moment où l'ostéoclaste se détache de l'os. Des études ont montré que la fixation de l'ostéoclaste sur l'os est médiée par des récepteurs : les intégrines. Les intégrines sont une superfamille de récepteurs médiant les processus d'adhésion cellule/cellule et plus particulièrement cellule/matrice, incluant notamment α_{IIb}β₃ comme récepteur des plaquettes sanguines (fibrinogène) et αᵥβ₃ comme récepteur de la vitronectine.

Les peptides contenant le motif RGD ainsi que les anticorps anti αᵥβ₃ sont connus pour leur capacité d'inhibition de la résorption de la dentine, d'empêchement de l'adhésion des ostéoclastes sur les matrices minéralisées (Horton et al. Exp. Cell. Res. (1991), 195, 368). Le peptide Echistatine isolé du venin de serpent contenant également un motif RGD et décrit comme inhibiteur de l'adhésion des ostéoclastes à l'os est donc un puissant inhibiteur de la résorption osseuse dans les tissus en culture in vitro (Sato et al. J. Cell. Biol. (1990), 111, 1713) et in vivo chez le rat (Fisher et al. Endocrinology (1993) 132, 1411).

Le récepteur αᵥβ₃ est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés de formule (I) selon l'invention.

En effet, les récepteurs αᵥβ₃, exprimés au niveau de la membrane des ostéoclastes sont à la base du processus d'adhésion/résorption, contribuent à l'organisation du cytosquelette cellulaire, et sont impliqués dans l'ostéoporose. Les récepteurs αᵥβ₃ exprimés au niveau des cellules du muscle lisse de l'aorte, stimulent leur migration vers la néointima, ce qui entraîne la formation de l'artériosclérose et la survenue de resténose post-angioplastique (Brown et al., cardiovascular Res. (1994), 28, 1815).

Les cellules endothéliales secrètent des facteurs de croissance qui sont mitogènes pour l'endothélium et peuvent contribuer à la formation de nouveaux vaisseaux sanguins (Angiogénèse). Les antagonistes de l'intégrin αᵥβ₃ peuvent ainsi entraîner une régression des tumeurs cancéreuses en induisant l'apoptose des vaisseaux sanguins angiogéniques. (Brook et al. Cell (1994) 79, 1157). Cheresh et al (Science 1995, 270, 1500) ont décrit des anticorps anti-αᵥβ₃ ou des antagonistes du récepteur αᵥβ₃ qui inhibent le processus d'angiogénèse induit par bFGF dans l'oeil de rat, une propriété qui peut être utilisée pour le traitement des rétinopathies, notamment du diabétique.

La demande de brevet WO-A-94/12181 décrit des systèmes aromatiques ou non aromatiques substitués et WO-A-94/08577 décrit des hétérocycles substitués comme antagonistes du récepteur de fibrinogène et inhibiteurs de l'agrégation plaquettaire. EP-A-528 586 et EP-A-528587 décrivent des dérivés de la phénylalanine substitués par un aminoalkyle ou un hétérocycle et WO-A-95/32710 décrit des dérivés aryliques comme inhibiteurs de la résorption osseuse par les ostéoclastes. WO-A-96/00574 décrit des benzodiazépines et WO-A-96/00730 décrit des composés inhibiteurs du récepteur fibrinogène, en particulier benzodiazépines qui sont liés à un cycle à 5 chaînons azoté comme antagonistes du récepteur de la vitronectine. DE-A-19654483 décrit des antagonistes du récepteur de la vitronectine dérivés de la tyrosine. DE-A-19629816.4 revendique des dérivés de cycloalkyles comme antagonistes du récepteur de la vitronectine. D'autres investigations ont permis de montrer que les dérivés d'acylguanidine de formule (I) présentent une forte activité comme inhibiteur du récepteur de la vitronectine et de la résorption osseuse médiée via les ostéoclastes.

L'invention a pour objet les composés de formule (I) 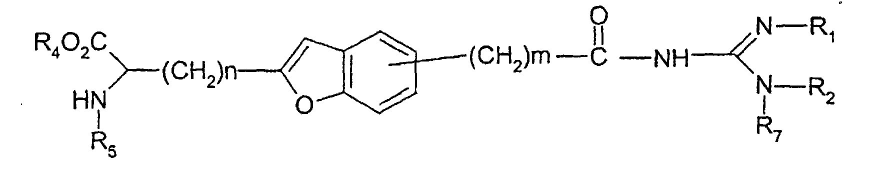 dans laquelle soit R₁ et R₂, indépendamment l'un de l'autre, représentent un atome d'hydrogène ou un groupement alkyle renfermant de 1 à 6 atomes de carbone non substitué ou substitué par R₃,
soit R₁ et R₂ forment ensemble un radical alkylène bivalent renfermant de 2 à 9 atomes de carbone, saturé ou insaturé, tel que -(CH₂)ₚ- dans lequel p est 2, 3, 4, 5, 6, 7, 8 ou 9, non substitué ou substitué par un ou plusieurs radicaux choisis parmi halogène, (C₁-C₆)-alkyle, (C₁-C₆)-alkoxy, (C₆-C₁₄)-aryle, (C₆-C₁₄)-aryle- (C₁-C₆)-alkyle, (C₅-C₁₄)-hétéroaryle, (C₅-C₁₄)-hétéroaryle-(C₁-C₆)-alkyle, (C₃-C₁₂)-cycloalkyle, (C₃-C₁₂)-cycloalkyle (C₁-C₆)-alkyle et oxo, ledit radical alkylène bivalent pouvant être accolé au niveau de la liaison carbone-carbone à un carbocycle ou un hétérocycle de 5 à 7 chaînons, renfermant 1 ou 2 atomes d'azote, saturé ou insaturé, non substitué ou substitué par 1 ou 2 radicaux R₃
R₃ représente un groupement (C₁-C₈)-alkyle, (C₁-C₈)-alkoxy,
(C₆-C₁₄)-aryle, (C₆-C₁₄)-aryle-(C₁-C4)-alkyle, (C₅-C₁₄)-hétéroaryle, (C₅-C₁₄)-hétéroaryle-(C₁-C₄)-alkyle, halogène, trifluorométhyle, hydroxyle, nitro, amino, NH-((C₁-C₄)-alkyle), N((C₁-C₄)alkyle)₂, NHCO-(C₁-C₄)-alkyle ou CO-(C₁-C₄)alkyle ;
R₄ représente
soit un atome d'hydrogène,
soit un groupement (C₁-C₆)-alkyle-CO-O-(C₁-C₄)-alkyle ou (C₁-C₆)-alkyle, non substitué ou substitué par un radical choisi parmi hydroxyle, (C₁-C₄)-alkoxyl (C₁-C₄)-alkyl-SO₂, NR₉R₉' et N⁺R₉R₉'R₉"Q⁻, dans lesquels R₉, R₉' et R₉" indépendamment les uns des autres, représentent un hydrogène, un groupement (C₁-C₆)-alkyle, (C₆-C₁₄)-aryle, (C₆-C₁₄)-aryle-(C₁-C₄)-alkyle, (C₅-C₁₄)-hétéroaryle, (C₅-C₁₄)-hétéroaryle-(C₁-C₄)-alkyle et Q⁻ est un anion physiologiquement acceptable,
soit un des radicaux suivants : 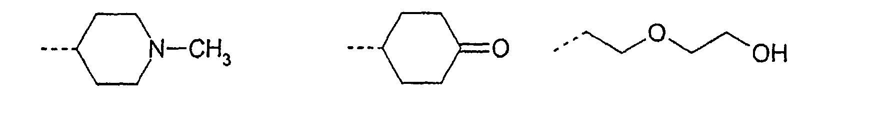 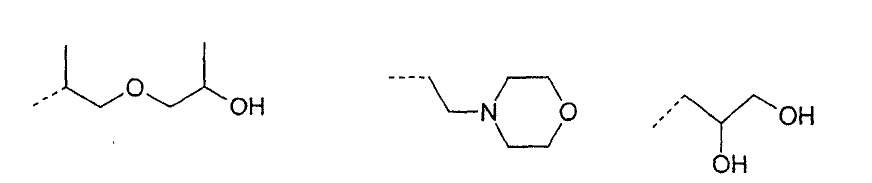 les traits en pointillés représentant la position de la liaison ;
R₅ représente un atome d'hydrogène ou un groupement choisi parmi COR₆, CO₂R₆, SO₂R₆, SO₂NHR₆, SO₂NHCOR₆, SO₂NHCO₂R₆, CONH₂ et CONHR₆ dans lesquels R₆ représente (C₁-C₈)-alkyle, (C₆-C₁₄)-aryle, (C₆-C₁₄)-aryle- (C₁-C₆)-alkyle, (C₅-C₁₄)-hétéroaryle ou (C₅-C₁₄)-hétéroaryle-(C₁-C₆)-alkyle, (C₃-C₂₀)(mono-, bi- ou tri-)-cycloalkyle, (C₃-C₂₀)(mono-, bi- ou tri-)-cycloalkyle-(C₁-C₆)alkyle, le radical aryle ou hétéroaryle étant non substitué ou substitué par 1, 2 ou 3 radicaux R₃ ;
R₇ représente un atome d'hydrogène, (C₁-C₆)-alkyle-O-CO-, hydroxyle, (C₁-C₆)-alkyle-O-CO-O- ou nitro ;
m est égal à 0, 1, 2 ou 3 ;
n est un entier égal à 1, 2 ou 3 ;
lesdits composés de formule (I) étant sous toutes leurs formes stéréo isomères possibles, seuls ou en mélange dans un rapport quelconque, le groupement acylguanidine adjacent au phényle étant en position para ou méta de l'oxygène, ainsi que leurs sels physiologiquement acceptables et leurs promédicaments (prodrugs).

Tous les radicaux qui peuvent se retrouver plusieurs fois dans les composés de formule (I), par exemple le radical R₃, sont indépendants les uns des autres et peuvent être identiques ou différents.
Les radicaux alkyles peuvent être linéaires ou ramifiés, saturés ou mono- ou poly-insaturés. Ceci s'applique également lorsqu'ils portent un substituant ou lorsqu'ils sont inclus dans des groupements tels que par exemple alkoxy, alkoxycarbonyle, aralkyle ou hétéroarylalkyle.
Par (C₁-C₈)-alkyle on entend les radicaux méthyle, éthyle, propyle, butyle, pentyle, hexyle, heptyle, octyle, nonyle, les n-isomères de ces radicaux, isopropyle, isobutyle, isopentyle, néopentyle, isohexyle, 3-méthylpentyle, 2,3,4-triméthylhexyle, sec-butyle, tert-butyle, tert-pentyle. Parmi les radicaux préférés on peut citer méthyle, éthyle, n-propyle, isopropyle, n-butyle, isobutyle, et tert-butyle.
Les radicaux alkylènes bivalents correspondant aux radicaux monovalents cités plus haut sont par exemple les radicaux méthylène, éthylène, 1,3-propylène, 1,2-propylène (=1-méthyléthylène), 2,3-butylène (=1,2-diméthyléthylène), 1,4-butylène, ou 1,6-hexylène. Les radicaux alkyles insaturés sont par exemple les radicaux alkényles tels que vinyle, 1-propényle, allyle, butényle, 3-méthyl-2-butényle ou les radicaux alkynyles tels que éthynyle, 1-propynyle ou propargyle.

Par radicaux alkylène bivalent insaturés on entend les radicaux alkénylène et alkynylène qui peuvent également être linéaires ou ramifiés. Il s'agit par exemple des radicaux vinylène, propénylène, éthynylène ou propynylène.
Les radicaux cycloalkyles peuvent être monocycliques, bicycliques ou tricycliques. Il s'agit par exemple des radicaux cyclopropyle, cyclobutyle, cyclopentyle, cyclohexyle, cycloheptyle, cyclooctyle, cyclononyle, cyclodecyle, cycloundecyle, cyclododécyle, cyclotétradécyle ou cyclooctadécyle qui le cas échéant peuvent être substitués par un ou plusieurs alkyles renfermant de 1 à 4 atomes de carbone. Comme radicaux cycloalkyles substitués, on peut citer le 4-méthylcyclohexyle et le 2,3-diméthylcyclohexyle.

Les radicaux bicycloalkyles et tricycloalkyles peuvent être non substitués ou substitués en toute position par un ou plusieurs groupes oxo et/ou 1 ou plusieurs groupes alkyles identiques ou différents tels que méthyle ou isopropyle et de préférence méthyle. La liaison de jonction du radical bi ou tricyclique peut se situer en toutes positions de la molécule. La liaison peut se situer au niveau de l'atome de carbone ponté ou d'un des autres atomes de carbone. Cette liaison peut présenter également toute position du point de vue de la stéréochimie, par exemple exo ou endo. Comme exemples de radicaux bicycloalkyles ou tricycloalkyles, on peut citer le camphanyle, le bornyle, l'adamantyle tels que le 1-adamantyle ou le 2-adamantyle, le caranyle, l'épiisobornyle, l'épibornyle, le norbornyle ou le norpinanyle.
Par halogène on entend fluor, chlore, brome ou iode.

Parmi les radicaux(C₆-C₁₄)-aryle carbocycliques, on peut citer le phényle, le naphtyle,le biphénylyle, l'anthryle ou le fluorényle et tout particulièrement le 1-naphtyle, le 2-naphtyle et le phényle.
Sauf indication contraire, les radicaux aryles, en particulier phényle, peuvent être non substitués ou substitués par un ou plusieurs radicaux identiques ou différents choisis parmi (C₁-C₈)-alkyle, en particulier (C₁-C₄)alkyle, (C₁-C₈)-alkoxy, halogène tel que fluor, chlore et brome, nitro, amino, trifluorométhyle, hydroxyle, méthylènedioxy, cyano, hydroxycarbonyle, aminocarbonyle, (C₁-C₄)-alkoxycarbonyle, phényle, phénoxy, benzyle et benzyloxy. En général, au plus 2 groupes nitro peuvent être utilisés dans les composés de formule (I) selon l'invention.

Dans le cas du phényle monosubstitué, le substituant peut être situé en position 2, 3 ou 4, et de préférence en position 3 ou 4. Dans le cas ou le phényle est disubstitué, les substituants peuvent être en position 2, 3 ou 2, 4 ou 2, 5 ou 2, 6 ou 3, 4 ou 3, 5. De préférence, dans les phényles disubstitués, les deux substituants sont en position 3,4. Lorsque ce phényle est trisubstitué les positions sont les suivantes : 2, 3, 4 ou 2, 3, 5 ou 2, 3, 6 ou 2, 4, 5 ou 2, 4, 6 ou 3, 4, 5. De la même manière, les radicaux naphtyles ou d'autres radicaux aryles peuvent être substitués en toutes positions, par exemple le radical 1-naphtyle en position 2-, 3-, 4-, 5-, 6-, 7-, et 8 et le radical 2-naphtyle en position 1-, 3-, 4-, 5-, 6-, et 7.

Le groupement (C₅-C₁₄)-hétéroaryle peut représenter un système aromatique monocyclique ou polycyclique dans lequel 1, 2, 3, 4 ou 5 atomes de carbone du cycle sont remplacés par des hétéroatomes, en particulier identiques ou différents du groupe constitué de l'azote, l'oxygène et le soufre. Parmi les groupements (C₅-C₁₄)-hétéroaryle on peut citer les groupements 2-pyridyle, 3-pyridyle, 4-pyridyle, pyrrolyle, furyle, thiényle, imidazolyle, pyrazolyle, oxazolyle, isoxazolyle, thiazolyle, isothiazolyle, tétrazolyle, pyridyle, pyrazinyle, pyrimidinyle, indolyle, isoindolyle, indazolyle, phtalazinyle, quinolyle, isoquinolyle, quinoxalinyle, quinazolinyle, cinnolinyle, β-carbolinyle ou encore des dérivés benzocondensés, cyclopenta- cyclohexa- ou cyclohepta- condensés de ces radicaux. Le système hétérocyclique peut être substitué par les mêmes substituants cités plus haut pour le système carbocyclique.

Parmi les radicaux hétéroaryles, sont préférés les systèmes aromatiques monocycliques ou bicycliques ayant 1, 2 ou 3 hétéroatomes, en particulier 1 ou 2 hétéroatomes, choisis parmi N, O ou S, et qui sont non substitués ou substitués par les groupes tels que (C₁-C₆)-alkyle, (C₁-C₆)-alkoxy, fluor, chlore, nitro, amino, trifluorométhyle, hydroxyle, (C₁-C₄)-alkoxycarbonyle, phényle, phénoxy, benzyloxy, et benzyle. Tout particulièrement, on peut citer les systèmes aromatiques monocycliques ou bicycliques renfermant de 5 à 10 chaînons ayant de 1 à 3 hétéroatomes, en particulier 1 ou 2 hétéroatomes, choisis parmi N, O et S et qui peuvent être substitués par 1 ou 2 substituants tels que (C₁-C₄)-alkyle, (C₁-C₄)-alkoxy, phényle, phénoxy, benzyle et benzyloxy.

Lorsque R₁ et R₂ forment ensemble un radical alkylène bivalent renfermant de 2 à 9 atomes de carbone, R₁ et R₂ forment ensemble, avec les deux atomes d'azote auxquels ils sont liés et l'atome de carbone central de la guanidine à laquelle les deux azotes sont liés, un monocyclique 1,3-diazahétérocycle qui est lié à l'atome d'azote dans le groupement (CH₂)m-CO-NH via sa position 2.
Comme exemple de 1,3-hétérocycles qui peuvent être substitués comme indiqués au niveau du radical (C₂-C₉)-alkylène ou de l'atome d'azote de la guanidine, on peut citer le radical 2-imidazolyle, le radical 4,5-dihydro-2-imidazolyle, le radical 1,4,5,6-tetrahydro-2-pyrimidinyle ou le radical 4,5,6,7-tétrahydro-lH-1,3-diazepin-2-yle.

Dans le cas où un cycle de 5 à 7 chaînons est condensé au niveau de la liaison carbone-carbone du radical (C₂-C₉)-alkylène, alors R₁ et R₂ forment ensemble, avec les deux atomes d'azote auquel ils sont liés et l'atome de carbone central de la guanidine à laquelle les deux azotes sont liés, un hétérocycle bicyclique qui est lié à l'atome d'azote du groupe (CH₂)m-CO-NH et qui peut être substitué comme indiqué plus haut.
Les cycles de 5 à 7 chaînons condensés au niveau de la liaison carbone-carbone du radical (C₂-C₉)-alkylène peuvent être saturés, monoinsaturés, di-insaturés ou aromatiques ; il s'agit par exemple du cycopropane, du cyclohexane, du cyclohexène, du cyclohexadiène, du cycloheptane ou du benzène.

Parmi les sytèmes aromatiques bicycliques liés à l'atome d'azote du groupement (CH₂)m-CO-NH on peut citer le radical 1,3a,4,5,6,6a-hexahydro-1,3-diazapentalen-2-yle, le radical 1H-2-benzimidazolyle, le radical 3a,4,5,6,7,7a-hexahydro-1H-benzymidazol-2-yle, le radical 4,5,6,7-tétrahydro-1H-benzimidazol-2-yle, le radical 4,7-dihydro-1H-benzyimidazole-2-yle ou le radical 1H-imidazo[4,5-b]pyridin-2-yle.
Dans le cas où le cycle condensé est substitué et/ou le radical (C₂-C₉)-alkylène est substitué, ils sont de préférence mono- ou di-substitués indépendamment l'un de l'autre par un radical R₃ identique ou différent.
Dans le cas où R₁ et/ou R₂ sont des groupements alkyles substitués, ils sont de préférence mono- ou di-substitués indépendamment l'un de l'autre par un radical R₃ identique ou différent.
Les atomes de carbone optiquement actifs contenus dans les composés de formule (I) peuvent indépendamment les uns des autres présenter la configuration R ou la configuration S. Les composés de formule (I) peuvent être sous la forme d'énantiomères purs ou de diastéréoisomères purs ou sous la forme d'un mélange d'énantiomères, par exemple sous forme de racémates ou de mélanges de diastéréoisomères.

La présente invention a donc pour objet les énantiomères purs, les mélanges de ces énantiomères, les diastéréoisomères purs et les mélanges de ces diastéréoisomères.

L'invention comprend les mélanges de deux ou plus de deux stéréoisomères de formule (I) et tous les rapports de ces stéréoisomères au sein desdits mélanges.

Les composés de formule (I) peuvent le cas échéant être présent sous forme d'isomères E ou d'isomères Z. L'invention a donc pour objet les isomères E purs, les isomères Z purs et les mélanges E/Z selon un rapport quelconque.

L'invention comprend également toutes les formes tautomères des composés de formule (I), par exemple en ce qui concerne la forme représentée par la formule (I), on considère la forme dans laquelle l'acylguanidine est présente sous la forme d'un groupe -CO-N=C(NHR₁)-NR₂R₇, et toutes les autres formes qui diffèrent par la position différente de l'atom d'hydrogène.
Enfin l'invention comprend les différents régioisomères liés à la position para ou méta du groupement (CH₂)ₘ-CONH-C(=NR₁)(NR₂R₇) par rapport à l'oxygène du benzofurane. Les diastéréoisomères, incluant les isomères E/Z peuvent être séparés en isomères individuels, par exemple par chromatographie. Les racémates peuvent être séparés en deux énantiomères par des méthodes courantes telles que la chromatographie en phase chirale ou par des méthodes de résolution. Les sels physiologiquement acceptables des composés de formule (I) sont en particulier des sels pharmaceutiquement utilisables ou non toxiques ou physiologiquement utilisables.

Lorsque les composés de formule (I) renferment un groupe acide tel que carboxylique, il s'agit par exemple des sels de métaux alcalins ou alcalinaux terreux tels que les sels de sodium, de potassium, de magnésium, de calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyéthyl)amine.
Lorsque les composés de formule (I) contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoique, maléique, fumarique, tartarique, méthanesulfonique ou para toluène sulfonique.

Les composés de formule (I) qui comportent un groupe basique et un groupe acide, tel que par exemple guanidino et carboxylique, peuvent être présents sous forme de Zwiterions (bétaînes), qui sont également inclus dans la présente invention.
L'anion Q⁻ physiologiquement acceptable qui est contenu dans les composés de formule (I) quand R₄ est un radical alkyle substitué par un groupement ammonium chargé, est de préférence un anion monovalent ou un équivalent d'un anion polyvalent d'un acide organique ou inorganique non toxique, physiologiquement acceptable et en particulier pharmaceutiquement acceptable, par exemple l'anion ou un équivalent d'un anion d'un des acides cités plus haut utiles pour la formation des sels d'addition. Q⁻ peut être par exemple un des anions (ou équivalent d'anion) d'un groupe choisi parmi chlore, sulfate, phosphate, acétate, trifluoroacétate, citrate, benzoate, maléate, fumarate, tartrate, méthanesulfonate et paratoluènesulfonate.

Les sels des composés de formule (I) peuvent être obtenus par les méthodes ordinaires connues de l'homme du métier, par exemple en combinant un composé de formule (I) avec un acide organique ou inorganique ou une base dans un solvant ou un dispersant ou à partir d'un autre sel par échange de cation. ou d'anion.

L'invention inclut également tous les sels des composés de formule (I) qui, à cause de leur faible acceptabilité physiologique, ne sont pas directement utilisable comme médicament, mais sont utilisables comme produits intermédiaires pour mettre en oeuvre des modifications chimiques ultérieures au niveau des composés de formule (I) ou comme produits de départ pour la préparation de sels physiologiquement acceptables.

La présente invention inclut également tous les solvates des composés de formule (I) par exemples les hydrates, les solvates formé avec les alcools, et tous les dérivés des composés de formule (I), par exemple les esters, prodrugs et autres dérivés physiologiquement acceptables, ainsi que les métabolites des composés de formule (I).

L'invention a plus particulièrement pour objet les prodrugs des composés de formule (I) qui peuvent être transformés en composé de formule (I) in vivo dans les conditions physiologiques. Les prodrugs des composés de formule (I), à savoir les dérivés chimiquement modifiés des composés de formule (I) afin d'obtenir des propriétés améliorées de manière désirée, sont connus de l'homme du métier.
Pour avoir plus d'information sur le type de prodrug envisagées dans la présente invention, on peut citer les ouvrages suivants : Fleicher et al., Advanced Drug Delivery Review 19 (1996) 115-130 ; Design of prodrugs, H. Bundgaard, Ed., Elsevier, 1985 ; H. Bungaard, Drugs of the Future 16 (1991) 443 ; Saulnier et al. Bioorg. Med. Chem. Lett. 4 (1994) 1985 ; Safadi et al. Pharmaceutical Res. 10 (1993) 1350. Parmi les différentes prodrugs appropriées des composés de formule (I) on peut citer de préférence :
- les prodrugs sous forme d'esters des groupes carboxyliques, en particulier du groupe COOH, qui est présent lorsque R₄ dans COOR₄ est un atome d'hydrogène
- les prodrugs sous forme d'acyle et carbamate pour les groupes contenant un azote acylable tel que les groupes amino et en particulier guanidine. Dans les prodrugs acylés ou sous forme de carbamate, une ou plusieurs fois, par exemple deux fois, un atome d'hydrogène situé sur l'atome d'azote est remplacé par un groupe acyle ou carbamate. Parmi les groupes acyles ou carbamates préférés, on peut citer les groupes R₁₀CO-, R₁₁OCO-, dans lesquels R₁₀ est un hydrogène, (C₁-C₁₈)-alkyle, (C₃-C₁₄) -cycloalkyle, (C₃-C₁₄) cycloalkyle-(C₁-C₈)-alkyle, (C₅-C₁₄)-aryle, dans lequel 1 à 5 atomes de carbone peuvent être remplacés par des hétéroatomes tels que N,O,S ou (C₅-C₁₄)-aryle-(C₁-C₈)alkyle, dans lequel 1 à 5 atomes de carbone dans la partie aryle peuvent être remplacés par des hétéroatomes tels que N,O,S et R₁₁ a les mêmes valeurs que R₁₀ à l'exception d'hydrogène.

Dans les composés de formule (I), les radicaux R₁ et R₂ représentent de préférence un atome d'hydrogène ou forment ensemble un radical bivalent alkylène saturé ou insaturé renfermant de 2 à 5 atomes de carbone et en particulier de 2 à 4 atomes de carbone et tout particulièrement 2 ou 3 atomes de carbone, lequel radical alkylène est non substitué ou substitué par un ou deux radicaux identiques ou différents choisis parmi halogène, (C₁-C₆)-alkyle, (C₁-C₆)-alkoxy, (C₆-C₁₄)-aryle, (C₆-C₁₄)-aryle- (C₁-C₆)alkyle, (C₅-C₁₄)-hétéroaryle, (C₅-C₁₄)-hétéroaryle-(C₁-C₆)-alkyle, (C₃-C₁₂)-cycloalkyle et (C₃-C₁₂)-cycloalkyle-(C₁-C₆)-alkyle et oxo, ledit radical alkylène bivalent pouvant être accolé au niveau de la liaison carbone-carbone à un carbocycle ou un hétérocycle de 5 à 7 chaînons, renfermant 1 ou 2 atomes d'azote, saturé ou insaturé, non substitué ou substitué par 1 ou 2 radicaux R₃.

Parmi les composés de formule (I), R₁ et R₂ représentent de préférence un atome d'hydrogène ou un groupe -(CH₂)ₚ-, dans lequel p est 2, 3, 4 ou 5, de préférence 2, 3 ou 4, tout particulièrement 2 ou 3, et qui est non substitué ou substitué par un ou deux radicaux identiques ou différents choisi parmi halogène, (C₁-C₆)-alkyle, (C₁-C₆)-alkoxy, (C₆-C₁₄)-aryle, (C₆-C₁₄)-aryle- (C₁-C₆)alkyle, (C₅-C₁₄)-hétéroaryle, (C₅-C₁₄)-hétéroaryle-(C₁-C₆)-alkyle, (C₃-C₁₂)-cycloalkyle et (C₃-C₁₂)-cycloalkyle-(C₁-C₆)-alkyle et oxo,, ledit radical -(CH₂)ₚ- pouvant être accolé au niveau de la liaison carbone-carbone à un carbocycle ou un hétérocycle de 5 à 7 chaînons, renfermant 1 ou 2 atomes d'azotes, saturé ou insaturé, non substitué ou substitué par 1 ou 2 radicaux R₃.
R₃ est de préférence un groupement alkyle ou alkoxy renfermant de 1 à 4 atomes de carbone.
R₄ est de préférence un atome d'hydrogène ou un groupement alkyle renfermant de 1 à 6 atomes de carbone non substitué ou substitué par un groupe choisi parmi (C₁-C₄)-alkoxy, (C₁-C₄)-alkyle-S(O)₂ et NR₉R₉' dans lequel R₉ et R₉' indépendamment l'un de l'autre représentent un atome d'hydrogène ou (C₁-C₄)-alkyle. R₄ est tout particulièrement un atome d'hydrogène ou un groupement alkyle renfermant de 1 à 4 atomes de carbone non substitué ou substitué par les radicaux cités plus haut. R₅ est de préférence un atome d'hydrogène, un groupement CO₂R₆, SO₂R₆, SO₂NHR₆ ou SO₂NHCO₂R₆
R₆ est de préférence un radical (C₁-C₈)-alkyle ou naphtyle, non substitué ou substitué par R₃, un radical (mono-, bi- ou tri-)-cycloalkyle renfermant de 3 à 12 atomes de carbone ou (C₃-C₁₂) (mono-, bi- ou tri-)-cycloalkyle-(C₁-C₆)-alkyle ou le radical de formule (II) 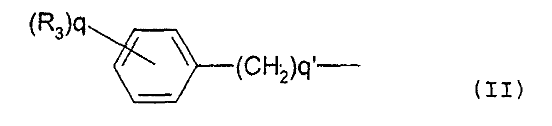 dans lequel les radicaux R₃ peuvent être identiques ou différents, et peuvent être localisés à n'importe quelle position du radical phényle, q est égal à 0, 1, 2 ou 3, de préférence 0 ou 1 et tout particulièrement 0 et q' est égal à 0 ou 1. R₆ représente plus particulièrement un radical alkyle renfermant de 1 à 4 atomes de carbone, un radical phényle mono, bi ou trisubstitué par (C₁-C₆)-alkyle, un radical naphtyle, un radical adamantylméthyle ou le radical de formule (II) dans lequel q est 0 ou 1. R₆ représente tout particulièrement le radical de formule (II) avec q égal à 0 ou 1 et q' égal à 1, c'est à dire un radical benzyle non substitué ou monosubstitué en position ortho, méta ou para par R₃.
R₇ est de préférence un atome d'hydrogène ou un groupement alkyloxycarbonyle renfermant de 2 à 7 atomes de carbone, plus particulièrement hydrogène ou alkyloxycarbonyle renfermant de 2 à 5 atomes de carbone et tout particulièrement hydrogène. Les composés préférés de formule (I) sont les composés dans lesquels un ou plusieurs radicaux ont les significations préférées.
En particulier, l'invention a pour objet un composé de formule (I') 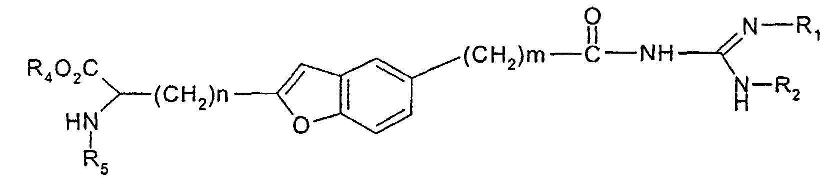 dans laquelle, R₁ et R₂ représentent un atome d'hydrogène ou forment ensemble un radical bivalent alkylène saturé ou insaturé renfermant de 2 à 5 atomes de carbone ou plus particulièrement représentent un atome d'hydrogène ou forment ensemble un groupe -(CH₂)ₚ-, dans lequel p est 2, 3, 4 ou 5, de préférence 2, 3 ou 4, tout particulièrement 2 ou 3, ledit radical alkylène ou groupe -(CH₂)ₚ- étant non substitué ou substitué par un ou deux radicaux identiques ou différents choisi parmi halogène, (C₁-C₆)-alkyle, (C₁-C₆)-alkoxy, (C₆-C₁₄)-aryle, (C₆-C₁₄)-aryle-(C₁-C₆)alkyle, (C₅-C₁₄)-hétéroaryle, (C₅-C₁₄)-hétéroaryle-(C₁-C₆)-alkyle, (C₃-C₁₂)-cycloalkyle et (C₃-C₁₂)-cycloalkyle-(C₁-C₆)-alkyle et oxo, ledit alkylène ou groupe -(CH₂)ₚ- pouvant être accolé au niveau de la liaison carbone-carbone à un carbocycle ou un hétérocycle renfermant 1 ou 2 atomes d'azote, de 5 à 7 chaînons, saturé ou insaturé, non substitué ou substitué par R₃ et en particulier par 1 ou 2 radicaux R₃ ;
R₃ représente un groupement alkyle ou alkyloxy renfermant de 1 à 6 atomes de carbone ;
R₄ représente un atome d'hydrogène ou un groupement alkyle renfermant de 1 à 6 atomes de carbone non substitué ou substitué par un groupe choisi parmi (C₁-C₄)-alkoxy, (C₁-C₄)-alkyle-S(O)₂ et NR₉R₉' dans lequel R₉ et R₉' indépendamment l'un de l'autre représentent un atome d'hydrogène ou (C₁-C₄)-alkyle ;
R₅ représente un atome d'hydrogène, un groupement CO₂R₆, SO₂R₆, SO₂NHR₆ ou SO₂NHCO₂R₆ dans lesquels R₆ est un radical alkyle renfermant de 1 à 8 atomes de carbone, un radical naphtyle, non substitué ou substitué par (C₁-C₆)-alkyle, un radical cycloalkyle renfermant de 3 à 12 atomes de carbone ou (C₃-C₁₂)-cycloalkyle-(C₁-C₆)alkyle ou le radical de formule (II) 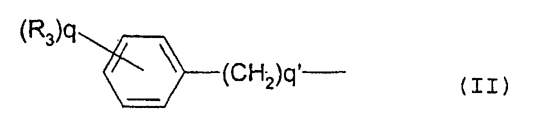 dans lequel les radicaux R₃ peuvent être identiques ou différents, et peuvent être localisés à n'importe quelle position du radical phényle, q et q' sont égaux à 0 ou 1 ;
m est un entier égal à 1, 2 ou 3 ;
n est un entier égal à 1, 2 ou 3 ;
lesdits composés de formule (I') étant sous toutes leurs formes stéréo isomères possibles, seuls ou en mélange selon un rapport quelconque, ainsi que leurs sels physiologiquement acceptables et leurs prodrugs.

L'invention a également plus particulièrement pour objet un composé de formule (I'), dans laquelle, R₁ et R₂ représentent un atome d'hydrogène ou forment ensemble un radical bivalent alkylène saturé ou insaturé renfermant de 2 à 4 atomes de carbone ou plus particulièrement représentent un atome d'hydrogène ou forment ensemble un groupe -(CH₂)ₚ-, dans lequel p est 2, 3 ou 4, ledit radical alkylène ou groupe -(CH₂)ₚ- étant non substitué ou substitué par un ou deux radicaux identiques ou différents choisi parmi halogène, (C₁-C₆)-alkyle, (C₁-C₆)-alkoxy, (C₆-C₁₄)-aryle, (C₆-C₁₄)-aryle-(C₁-C₆)alkyle, (C₅-C₁₄)-hétéroaryle, (C₅-C₁₄)-hétéroaryle-(C₁-C₆)-alkyle, (C₃-C₁₂)-cycloalkyle, (C₃-C₁₂)-cycloalkyle-(C₁-C₆)-alkyle et oxo, ledit alkylène ou groupe -(CH₂)ₚ pouvant être accolé au niveau de la liaison carbone-carbone à un carbo-cycle ou un hétérocycle renfermant 1 ou 2 atomes d'azotes, de 5 à 7 chaînons, saturé ou insaturé, non substitué ou substitué par R₃ et en particulier par 1 ou 2 radicaux R₃ ;
R₃ représente un groupement alkyle ou alkyloxy renfermant de 1 à 6 atomes de carbone ;
R₄ représente un atome d'hydrogène ou un groupement alkyle renfermant de 1 à 6 atomes de carbone ;
R₅ représente un atome d'hydrogène, un groupement CO₂R₆, SO₂R₆, SO₂NHR₆ ou SO₂NHCO₂R₆ dans lesquels R₆ est un radical (C₁-C₈)-alkyle ou naphtyle, non substitué ou substitué par R₃, un radical cycloalkyle renfermant de 3 à 12 atomes de carbone ou (C₃-C₁₂)-cycloalkyle-(C₁-C₆)-alkyle ou le radical de formule (II) 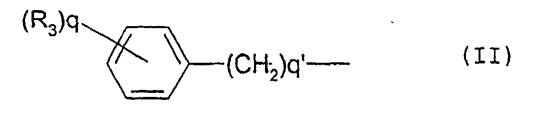 dans lequel les radicaux R₃ peuvent être identiques ou différents, et peuvent être localisés à n'importe quelle position du radical phényle, q et q' sont égaux à 0 ou 1 ;
m est un entier égal à 1, 2 ou 3 ;
n est un entier égal à 1, 2 ou 3 ;
lesdits composés de formule (I) étant sous toutes leurs formes stéréo isomères possibles, seuls ou en mélange selon un rapport quelconque, ainsi que leurs sels physiologiquement acceptables et leurs prodrugs.

L'invention a également plus particulièrement pour objet un composé de formule (I'), dans laquelle, R₁ et R₂ représentent un atome d'hydrogène ou forment ensemble un radical bivalent alkylène saturé ou insaturé renfermant de 2 à 3 atomes de carbone ou plus particulièrement représentent un atome d'hydrogène ou forment ensemble un groupe -(CH₂)ₚ-, dans lequel p est 2 ou 3, ledit radical alkylène ou groupe -(CH₂)ₚ-étant non substitué ou substitué par un ou deux radicaux identiques ou différents choisi parmi halogène, (C₁-C₆)-alkyle, (C₁-C₆)-alkoxy, (C₆-C₁₄)-aryle, (C₆-C₁₄) -aryle- (C₁-C₆)alkyle, (C₅-C₁₄)-hétéroaryle, (C₅-C₁₄)-hétéroaryle- (C₁-C₆)-alkyle, (C₃-C₁₂)-cycloalkyle, (C₃-C₁₂)-cycloalkyle-(C₁-C₆)-alkyle et oxo, ledit alkylène ou groupe -(CH₂)ₚ- pouvant être accolé au niveau de la liaison carbone-carbone à un carbocycle ou un hétérocycle renfermant 1 ou 2 atomes d'azotes, de 5 à 7 chaînons, saturé ou insaturé, non substitué ou substitué par R₃ et en particulier par 1 ou 2 radicaux R₃ ;
R₃ représente un groupement alkyle ou alkyloxy renfermant de 1 à 6 atomes de carbone ;
R₄ représente un atome d'hydrogène ou un groupement alkyle renfermant de 1 à 6 atomes de carbone ; R₅ représente un atome d'hydrogène, un groupement CO₂R₆, SO₂R₆, SO₂NHR₆ ou SO₂NHCO₂R₆ dans lesquels R₆ est un radical (C₁-C₈)-alkyle ou naphtyle, non substitué ou substitué par R₃, un radical cycloalkyle renfermant de 3 à 12 atomes de carbone ou (C₅-C₁₅)-cycloalkyle-(C₁-C₆)-alkyle ou le radical de formule (II) 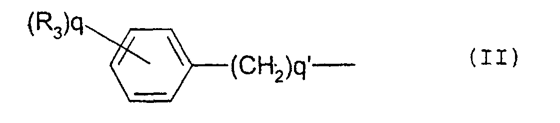 dans lequel les radicaux R₃ peuvent être identiques ou différents, et peuvent être localisés à n'importe quelle position du radical phényle, q et q' sont égaux à 0 ou 1 ;
m est un entier égal à 2 ;
n est un entier égal à 1 ;
lesdits composés de formule (I) étant sous toutes leurs formes stéréo isomères possibles, seuls ou en mélange selon un rapport quelconque, ainsi que leurs sels physiologiquement acceptables et leurs prodrugs.
Parmi les composés préférés de formule (I), se trouvent les composés dans lesquels le carbone asymétrique portant les groupements CO₂R₄ et NHR₅ est de configuration S.

L'invention a tout particulièrement pour objet les composés de formule (I) ou (I') tels que définis plus haut dans lesquels R₅ est un radical CO₂R₆, R₆ étant tel que défini plus haut et notamment -CH₂Ph, -C(CH₃)₃ et CH₂-Adamantyle, lesdits composés de formule (I) étant sous toutes leurs formes stéréo isomères possibles, seuls ou en mélange selon un rapport quelconque, ainsi que leurs sels physiologiquement acceptables et leurs prodrugs.

L'invention a tout particulièrement pour objet les composés de formule (I) ou (I') tels que définis plus haut dans lesquels R₅ est un radical SO₂R₆, R₆ étant tel que défini plus haut et notamment alkyle renfermant de 1 à 6 atomes de carbone, naphtyle et phényle substitué par un ou plusieurs radicaux alkyle renfermant de 1 à 6 atomes de carbone ou groupement CF₃ lesdits composés de formule (I) étant sous toutes leurs formes stéréo isomères possibles, seuls ou en mélange selon un rapport quelconque, ainsi que leurs sels physiologiquement acceptables et leurs prodrugs.

L'invention a tout particulièrement pour objet les composés de formule (I) ou (I') tels que définis plus haut dans lesquels R₅ est un radical SO₂NHR₆ ou SO₂NHCO₂R₆, R₆ étant tel que défini plus haut et notamment -CH₂Ph, -C(CH₃)₃ et CH₂-Adamantyle, lesdits composés de formule (I) étant sous toutes leurs formes stéréo isomères possibles, seuls ou en mélange selon un rapport quelconque, ainsi que leurs sels physiologiquement acceptables et leurs prodrugs.

L'invention a également pour objet les composés de formule (I) dont les noms suivent :
- Acide 5-[3-oxo-3-[(1,4,5,6-tétrahydro-2-pyrimidinyl)amino)-propyl]-.alpha.-[[(phénylméthoxy)carbonyl]amino]-2-benzofuranepropanoique,
- 5-[3-oxo-3-[(1,4,5,6-tétrahydro-2-pyrimidinyl)amino]propyl-.alpha.-[[(phénylméthoxy)carbonyl]amino]-2-benzofuranpropanoate de 1-méthyléthyle,
- 5-[3-oxo-3-[(1,4,5,6-tétrahydro-2-pyrimidinyl)amino]-propyl]-.alpha.-[[(phénylméthoxy)carbonyl]amino]-2-benzofu ranpropanoate d'éthyle,
- Acide 5-[3-oxo-3-[(1,4,5,6-tétrahydro-2-pyrimidinyl)amino]-propyl]-.alpha.-[[[(tricyclo[3.3.1.1^{3,7}]dec-1-yl)méthoxy]carbonyl]amino]-2-benzofuranpropanoique,
- Acide 5-[3-oxo-3-[(1,4,5,6-tétrahydro-2-pyrimidinyl)-amino]propyl]-.alpha.-[[[[[(tricyclo[2.3.1.1^{3,7}]dec-1-yl)méthoxy]carbonyl] amino] sulfonyl] amino] -2-benzofuranpropanoique,
- Acide 5-[3-oxo-3-[(1,4,5,6-tétrahydro-2-pyrimidinyl)amino]-propyl]-.alpha.-[[[[(phényl)méthoxy]carbonyl]-amino]-sulfonyl]amino]-2-benzofuranpropanoique,
- Acide.alpha.-[[[(phénylméthyl)-amino]sulfonyl]amino]-5-[3-oxo-3-[(1,4,5,6-tétrahydro-2-pyrimidinyl)amino]propyl]-2-benzofuranpropanoique,
- Acide .alpha.-[[[4-(1,1-diméthylethyl)phényl]-sulfonyl]-amino]-5-(3-oxo-3-[(1,4,5,6-tétrahydro-2-pyrimidinyl)amino]-propyl]-2-benzofuranpropanoique,
- Acide .alpha.-[[[4-(1-méthyléthyl)phényl]sulfonyl]amino]-5-(3-oxo-3-[(1,4,5,6-tétrahydro-2-pyrimidinyl)amino]propyl]-2-benzofuranpropanoique,
- Acide 5-[3-oxo-3-[(1,4,5,6-tétrahydro-2-pyrimidinyl)-amino]propyl]-.alpha.-[propylsulfonyl)amino]-2-benzofuranpropanoique,
- Acide.alpha.-[méthylsulfonyl)amino]-5-[3-oxo-3-[(1,4,5,6-tétrahydro-2-pyrimidinyl)amino]propyl]-2-benzofuranpropanoique,
- Acide.alpha.-[(1-naphtalenylsulfonyl)amino]-5-[3-oxo-3-[(1,4,5,6-tétrahydro-2-pyrimidinyl)amino]propyl]-2-benzofuranpropanoique,
- Alpha.-[(1-naphtalenylsulfonyl)amino]-5-[3-oxo-3-[(1,4,5,6-tétrahydro-2-pyrimidinyl)amino]propyl] -2-benzofuranpropanoate de 1-méthyléthyle,
ainsi que leurs sels physiologiquement acceptables et leurs prodrugs.

La présente invention a également pour objet un procédé de préparation des composés de formule (I). Les composés peuvent généralement être préparés, par exemple au cours d'une synthèse convergente par couplage de deux ou plusieurs fragments qui peuvent être dérivés par rétrosynthèse des composés de formule (I). Afin d'éviter que les groupes fonctionnels puissent mener à des réactions indésirables ou secondaires au cours de chaque étape de synthèse, il peut être avantageux ou nécessaire au cours de la synthèse des composés de formule (I), d'introduire les groupes fonctionnels sous forme de précurseurs qui sont par la suite convertis en groupes fonctionnels désirés ou de bloquer temporairement ces groupes fonctionnels en mettant en oeuvre une stratégie de groupe protecteur appropriée à la synthèse qui est connue par l'homme de l'art (Greene, Wuts protective Group in Organic Synthesis, Wiley 1991).
Ainsi les composés de formule (I) peuvent être préparés, par exemple, en mettant en oeuvre les étapes suivantes
a) formation d'un acide carboxylique ou un dérivé d'acide carboxylique de formule (III) 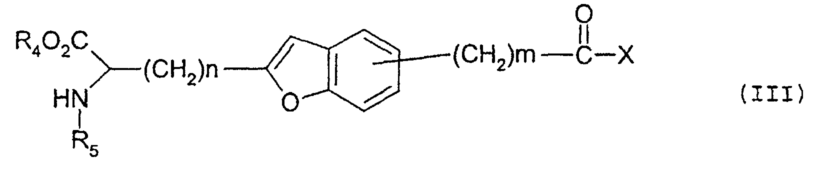 dans laquelle R₄, R₅, n et m sont tels que définis plus haut pour la formule (I) et dans laquelle X est un groupe partant substituable par un nucléophile,
   par action de l'aminoester de formule (V) 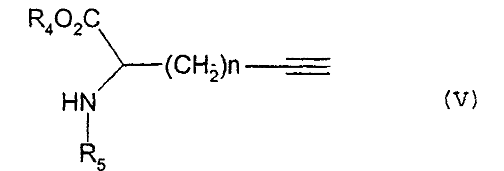 dans laquelle R₄, R₅, et n sont tels que définis plus haut pour la formule (I),
   avec un acide carboxylique ou un dérivé d'acide carboxylique de formule (VI), en présence d'un catalyseur et en milieu basique 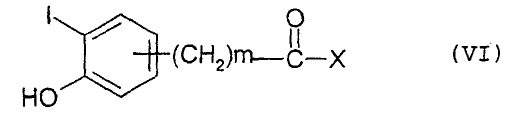 dans laquelle m et X sont tels que définis plus haut, les groupes fonctionnels, éventuellement présents sous forme de précurseurs ou sous forme protégée, étant par la suite convertis en groupes présents dans les composés de formule (I) et
b)couplage de l'acide carboxylique ou du dérivé d'acide carboxylique de formule (III) telle que définie plus haut avec une guanidine ou un dérivé de la guanidine de formule (IV) 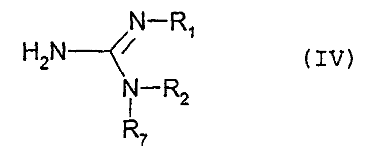 dans laquelle R₁, R₂ et R₇ sont tels que définis dans la formule (I), les groupes fonctionnels éventuellement présents sous forme de précurseur ou sous forme protégée étant par la suite convertis en groupes présents dans les composés de formule (I).
   La formation d'un acide carboxylique ou d'un dérivé d'acide carboxylique de formule (III) par action de l'amino ester de formule (V) avec acide carboxylique ou un dérivé d'acide carboxylique de formule (VI) s'effectue de préférence en présence d'un oxyde de cuivre tel que le Cu₂O dans un solvant tel que la pyridine.

Le groupement COX dans la formule (III) ou (VI) est de préférence le groupe acide carboxylique ou un dérivé activé de l'acide carboxylique. X, par exemple est hydroxyle ou halogène, en particulier, chlore ou brome, alkoxy, de préférence méthoxy ou éthoxy, aryloxy, par exemple phénoxy, ou pentafluorophénoxy, phénylthio, méthylthio, 2-pyridylthio ou un hétérocycle azoté lié via un atome d'hydrogène, en particulier un azole tel que par exemple 1-imidazolyle. X peut être également par exemple ((C₁-C₄)-alkyle)-O-CO-O- ou tolylsulfonyloxy et le dérivé d'acide activé peut être un anhydride mixte.
Si X est un hydroxyle, donc si la guanidine de formule (IV) réagit avec un acide carboxylique de formule (III), alors l'acide carboxylique est d'abord activé. L'activation peut être effectuée par exemple avec le dicyclohexylcarbodiimide (DCCI) ou avec le O-((cyano(éthoxycarbonyl)-méthylène)amino)-1,1,3,3-tetraméthyluronium tetrafluoroborate (TOTU ; König et al, Proc. 21st Europ. Peptide Symp. 1990 (Eds Giralt, Andreu), Escom, Leiden 1991, p.243) ou d'autre agent activant courant dans la synthèse peptidique.
Outre les guanidine libres de formule (IV), les sels de guanidine peuvent également être mis en oeuvre dans la réaction avec les composés de -formule (III), les guanidine libres étant formées insitu ou par une étape séparée au moyen d'une base.

La réaction d'un dérivé activé d'acide carboxylique de formule (III) avec la guanidine (ou dérivé) de formule (IV) est de préférence effectuée d'une manière connue en soi dans un solvant organique protique ou aprotique, mais inerte. Dans ce cas, on utilise des solvants tels que le méthanol, l'isopropanol, le tert-butanol, le diméthylformamide ou le tétrahydrofurane à des températures allant de 0°C à la température de reflux de ces solvants, notamment lors de la réaction des esters méthyliques ou éthyliques (X est un méthoxy ou un éthoxy) avec les guanidines.

Les réactions des composés de type COX avec les guanidines libres sont avantageusement mises en oeuvre dans un solvant aprotique inerte tel que le diméthylformamide, le tétrahydrofurane, le diméthoxyéthane ou le dioxane, le cas échéant en additionnant une base telle que par exemple le tert-butoxide de potassium ou le méthoxide de sodium. Cependant, l'eau peut également être utilisée comme solvant dans les réactions des composés de formule (III) avec les guanidines de formule (IV), par exemple en utilisant une base telle que l'hydroxyde de sodium.
Si X est le chlore, la réaction sera de préférence menée par addition d'un piégeur d'acide, par exemple d'une base ou d'un excès de guanidine (ou dérivé). Le mélange réactionnel est ensuite traité et si désiré le produit réactionnel est purifié selon les méthodes connues de l'homme du métier.

Les groupes protecteurs éventuellement présents dans les composés obtenus à partir des composés de formules (V), (VI), (III) et (IV) sont ensuite éliminés par des procédés classiques, par exemple les groupes tert-butyle ester sont convertis en acide carboxylique par traitement avec de l'acide trifluoroacétique, les groupes benzyles sont éliminés par hydrogénation ou encore les groupes fluorénylméthoxycarbonyle sont éliminés en présence d'amine secondaire et d'autres réactions sont mises en oeuvre par des procédés standards, par exemple par des réactions d'acylation. Si nécessaire, la conversion en des sels physiologiquement acceptables s'effectue par des procédés connus de l'homme du métier.

Lorsque R₅ représente un atome d'hydrogène, la fonctionnalisation de l'amine en groupe présent dans les composés de formule (I), c'est à dire en particulier lorsque R₅ représente un groupement CO₂R₆, SO₂R₆, SO₂NHR₆ ou SO₂NHCO₂R₆ s'effectue au niveau des composés de formule (V), (III) ou (I) et de préférence (III). A titre d'exemple pour obtenir les composes de formule (III)avec R₅=CO₂R₆ à partir de l'amine correspondante on fait réagir un composé de formule X'-CO₂R₆, X' étant un groupe partant et notamment O-succinique ou encore un halogène. Pour obtenir les composés de formule (III) avec R₅=SO₂R₆ à partir de l'amine correspondante on fait réagir un composé de formule R₆SO₂X', X'étant notamment un halogène.
pour obtenir les composés de formule (III) avec R₅=SO₂NHCO₂R₆ à partir de l'amine correspondante on fait réagir un composé de formule X'SO₂NHCO₂R₆, X'étant notamment un halogène ou de préférence par action d'un isocyanate de formule ClSO₂NCO en présence d'un alcool R₆OH.
Enfin pour obtenir les composés de formule (III) avec R₅=SO₂NHR₆ à partir de l'amine correspondante on fait réagir d'abord un isocyanate du type ClSO₂NCO en présence d'un alcool terbutylique, puis un halogénure de formule R₆X et enfin un agent de déprotection du groupement BOC.

Les composés de formule (IV) sont commerciaux ou aisément accessibles à l'homme du métier.
Les composés de formule (VI) sont également connus ou également aisément accessible à l'homme du métier, selon notamment les méthodes décrites plus bas à la préparation 3.

Les composés de formule(V) peuvent être préparés selon des procédés décrits dans la demande internationale WO97/40052 ou encore sont accessibles par analogie. Ce procédé est illustré dans le schéma décrit plus bas, étant entendu que la présente invention n'est pas restreinte à ces synthèses ou ces produits de départ. Il n'y a pas de difficulté majeure pour l'homme du métier de prévoir des modifications des synthèses décrites dans notre demande pour la préparation d'autres composés de formule (I) selon l'invention.

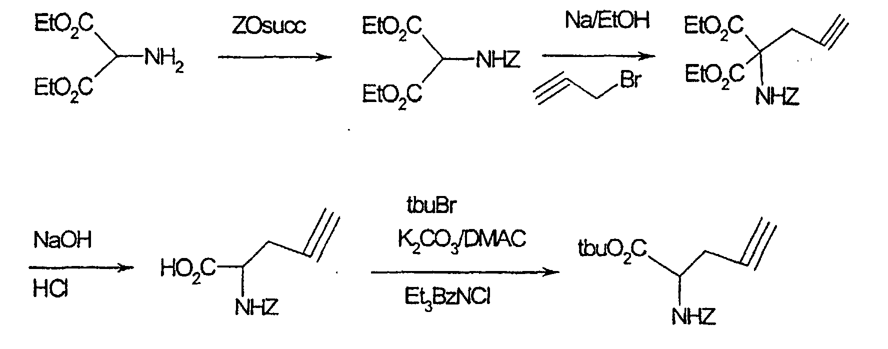

Les composés de formule (I) sont des composés ayant une activité pharmacologique et peuvent ainsi être utilisés à titre de médicaments dans le traitement ou la prévention des maladies de l'os, des maladies tumorales ainsi que des désordres cardiovasculaires.

Les composés de formule (I) ainsi que leurs sels physiologiquement acceptables et leurs prodrugs peuvent être administrés aux animaux, de préférence aux mammifères et en particulier aux êtres humains comme médicaments à titre thérapeutique ou prophylactique.
Ils peuvent être administrés tels quels ou en mélange avec un ou plusieurs autres composés de formule (I) ou encore sous la forme d'une préparation pharmaceutique (composition pharmaceutique) qui permet une administration entérale ou parentérale et qui, renferme à titre de composé actif une dose efficace d'au moins un composé de formule (I) et/ou ses sels physiologiquement acceptables et/ou ses prodrugs ainsi que des supports et/ou additifs courants et pharmaceutiquement inertes.

La présente invention a donc pour objet les composés de formule (I) et/ou leurs sels physiologiquement acceptables et/ou leurs prodrugs à titre de médicament.

La présente invention a également pour objet l'utilisation des composés de formule (I) et/ou leurs sels physiologiquement acceptables et/ou leurs prodrugs pour la préparation de médicaments destinés à la prévention ou au traitement des maladies citées plus haut ou plus bas, par exemple pour le traitement ou la prévention des maladies de l'os.

La présente invention a également pour objet les compositions pharmaceutiques qui permettent une administration entérale ou parentérale et qui renferment, à titre de composé actif, une dose efficace d'au moins un composé de formule (I) et/ou ses sels physiologiquement acceptables et/ou ses prodrugs ainsi qu'un ou plusieurs supports usuels et pharmaceutiquement inertes et le cas échéant un ou plusieurs additifs.
Les médicaments peuvent être administrés oralement, par exemple sous forme de pilules, de comprimés, de comprimés enrobés, de pelliculés, de granules, de gélules et capsules molles, de solutions, de sirops, d'émulsion, de suspension ou de mélange d'aérosol.
L'administration peut cependant être effectuée par voie rectale, par exemple sous forme de suppositoire ou par voie parentérale, par exemple sous forme de solutions injectables, d'infusions, de microcapsule ou d'implants ou par voie percutanée, par exemple sous la forme de pommade, de solutions, de pigments ou de colorants ou par d'autres voies telles que sous la forme d'aérosol ou de spray nasal.
Les préparations pharmaceutiques selon l'invention sont préparées selon des méthodes connues en soi, des supports organiques ou inorganiques, pharmaceutiquement inertes étant additionnés aux composés de formule (I) et/ou leurs sels physiologiquement acceptables et/ou leurs prodrugs.
Pour la production de pilules, de comprimés, de comprimés enrobés et de capsule en gélatine dure, il est possible d'utiliser par exemple, du lactose, de l'amidon de maïs ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc.
Les supports convenables pour les microcapsules ou les implants sont par exemple les copolymères d'acide glyoxilique et d'acide lactique. Les préparations pharmaceutiques contiennent normalement de 0,5% à 90% en poids de composés de formule (I) et/ou leurs sels physiologiquement acceptables. En plus des principes actifs et des supports, les préparations pharmaceutiques peuvent contenir des additifs tels que, par exemple, des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants.
Elles peuvent également contenir deux ou plusieurs composés de formule (I) et/ou leurs sels physiologiquement acceptables et/ou leurs prodrugs. En outre, en plus d'au moins un ou plusieurs composés de formule (I) et/ou leurs sels physiologiquement acceptables et/ou leurs prodrugs, ils peuvent contenir au moins un ou plusieurs principes actifs utilisables à titre thérapeutique ou prophylactique.

Les compositions pharmaceutiques renferment normalement de 0,2 à 500mg, et de préférence de 1 à 200 mg de composé de formule (I) et/ou leurs sels physiologiquement acceptables et/ou leurs prodrugs.

Les composés de formule (I) sont tout particulièrement des antagonistes des récepteurs de la vitronectine et sont capables ainsi par exemple d'inhiber l'adhésion des ostéoclastes sur la surface de l'os et ainsi la résorption osseuse par les ostéoclastes.
L'action des composés de formule (I) peut être démontrée par exemple dans un test dans lequel l'inhibition de la liaison de la vitronectine aux cellules qui contiennent le récepteur de la vitronectine est déterminée. Des précisions sur ce test sont données plus bas.
Comme antagonistes du récepteur de la vitronectine, les composés de formule (I) et leurs sels physiologiquement acceptables et leurs prodrugs conviennent en général pour le traitement ou la prévention de maladies liées aux interactions entre les récepteurs de la vitronectine et leurs ligands, dans les processus d'interaction cellule-cellule ou cellule-matrice ou qui peuvent être influencés par l'inhibition des interactions de ce type, pour soulager ou guérir lorsqu'une inhibition des interactions de ce type est désirée.
Comme on l'a expliqué au début, une telle interaction joue un rôle important dans la résorption osseuse, dans l'angiogénèse ou dans la prolifération des cellules du muscle vasculaire lisse.
Les maladies de l'os dont le traitement ou la prévention nécessitent l'emploi des composés de formule (I), sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, et la maladie de Paget. En outre les composés de formule (I) peuvent être utilisés pour soulager, empêcher ou traiter les désordres de l'os qui sont causés par les traitements par les glucocorticoides, les thérapies liées à la prise de stéroides ou de corticostéroïdes ou par les déficiences d'hormones sexuelles mâles ou femelles. Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes.
A côté de cette utilisation comme inhibiteur de la résorption osseuse médiée via les ostéoclastes, les composés de formule (I) et leurs sels physiologiquement acceptables et leurs prodrugs sont utilisés comme inhibiteurs de la croissance tumorale ou des métastases cancéreuses, dans le traitement des désordres inflammatoires, pour le traitement ou la prévention des désordres cardiovasculaires, tels que l'artériosclérose ou la resténose, ou le traitement ou la prévention de la nephropatie ou la rétinopathie telle que par exemple la rétinopathie diabétique.
Les composés selon l'invention peuvent posséder également une activité vis-à-vis d'autres intégrines qui interagissent avec leur ligand via la séquence tripeptidique RGD (αᵥβ₁, αᵥβ₅, α_{IIb}β₃), leur confèrent des propriétés pharmacologiques utilisables pour traiter les pathologies associées à ces récepteurs.
Cette activité vis-à-vis des intégrines rend ainsi les composés de formule (I) utilisables dans la prévention ou le traitement de nombreuses maladies telles que celles mentionnées plus haut ou dans la revue de Dermot Cox DN§P 8(4) Mai 1995, 197-205 dont le contenu est intégré dans la présente demande.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.
Dans le cas d'une administration par voie orale, la dose quotidienne varie en général de 0,01 à 100 mg/kg et de préférence de 0,1 à 50 mg/kg, en particulier de 0,1 à 5 mg/kg. Par exemple pour un adulte de 75 kg on pourra envisager une dose quotidienne variant de 0,3 à 0,5 mg/kg.
Dans le cas d'une administration par voie intraveineuse, la dose quotidienne varie approximativement de 0,01 à 100 mg/kg et de préférence de 0,05 à 10 mg/kg.
La dose quotidienne peut être divisée, en particulier dans le cas de l'administration de grande quantité de principe actif, en plusieurs, par exemple 2, 3 ou 4 parts. Le cas échéant, en fonction du comportement individuel, il peut être nécessaire d'administrer les différentes doses de manière croissante ou décroissante.
Mis à part l'utilisation des composés de formule (I) comme médicaments, on peut également envisager leur utilisation comme véhicule ou support de composés actifs afin de transporter ces composés actifs de manière spécifique vers un site d'action (Drug targeting, voir Targeted Drug Delivery, R.C. Juliano, Handbook of Experimental Pharmacology, Vol 100, Ed. Born, G.V.R. et al , Springer Verlag). Les composés actifs qui peuvent être transportés sont en particulier ceux utilisés pour le traitement ou la prévention des maladies citées plus haut.

Les composés de formule (I) et leurs sels peuvent également être employés comme agent de diagnostique, par exemple pour des méthodes in vitro ou comme auxiliaire dans des études biochimiques dans lesquelles on désire bloquer le récepteur de la vitronectine ou influencer les interactions cellules-cellules ou cellules-matrices. Ils peuvent en outre être utilisés comme intermédiaire pour la préparation d'autres composés, en particulier d'autres agents actifs, qui sont accessibles à partir des composés de formule (I), part exemple par modification ou introduction de radicaux ou de groupes fonctionnels.

### Exemples

Les produits ont été identifiés par spectre de masse (MS) infrarouge (IR) et/ou spectre RMN. Les composés, qui ont été purifiés par chromatographie en utilisant un éluant qui contient par exemple de l'acide acétique ou trifluoroacétique, et qui ensuite sont séchés ou dans lesquels lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

Abbrévations/noms chimiques éventuellement employés : PCC : pyridine chlorochorate DMF ; DMF : diméthylformamide ; THF : tétrahydrofurane ; MeOH : méthanol ; AcOEt acétate d'éthyle ; TFA :acide trifluoroacétique ; TEA : triéthylamine ; BTEAC : chlorure de benzyltriéthylammonium ; DMAC : diméthylacétamide ; ep. (Épaulement) ; F (fort) ; s (singulet) ; d (doublet) ; t (triplet) ; 1 (large) m (multiplet).

### Préparation 1 (P1)

### 5- (3-méthoxy-3-oxopropyl)-.alpha.[[(phénylméthoxy)carbonyl]amino]-2-benzofuranepropanoate de 1,1-diméthyléthyle

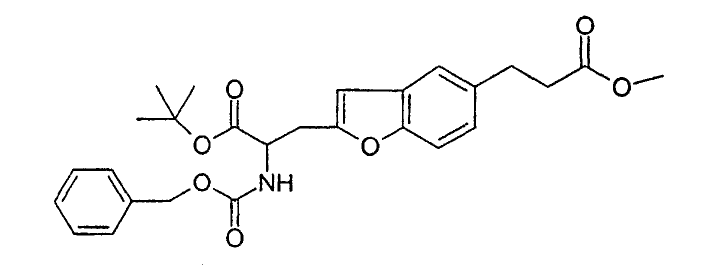

### Stade a : Alkylation

### 2-[[(phénylméthoxy)carbonyl]amino]-2-propynylpropanedioate de diéthyle

A 6 g de 2-[[(phénylméthoxy)carbonyl]amino]-propanedioate de diéthyle (préparé selon Bladon. C.M. J.Chem. Soc. Perkin. Trans 1 9, 1151-1158 (1990) dans 30 ml d'éthanol, on ajoute une solution d'éthylate de sodium préparée préalablement à partir de 500 mg de sodium dans 20 ml d'éthanol, puis après 30 minutes, 3 g de bromure de propargyle à 80 % dans le toluène et porte l'ensemble au reflux pendant 1 heure. On ramène ensuite à température ambiante, évapore sous pression réduite, reprend avec du dichlorométhane, lave, sèche et obtient après évaporation sous pression réduite 6 g du produit attendu que l'on purifie par chromatographie en éluant avec un mélange Cyclohexane/Acétate d'éthyle 85/15. On obtient 2,82 g de produit purifié.

### Stade b : Saponification + Décarboxylation

### Acide 2-[[(phénylméthoxy)carbonyl]amino]-4-pentynoique

A 4,5 g du produit obtenu au stade précédent dans 45 ml de méthanol, on ajoute 6,75 ml de soude et agite 4 heures, puis ramène le pH à 4,2 - 4,3 par ajout d'acide chlorhydrique 2N, extrait avec de l'acétate d'éthyle, lave, sèche et évapore sous pression réduite jusqu'à obtention de 4,7 g de produit brut (diacide intermédiaire). On ajoute ensuite 40 ml de dioxane, porte au reflux pendant 1 heure, puis évapore sous pression réduite jusqu'à obtention de 3,51 g de produit attendu décarboxylé.
**MS :** 246- = [M-H]⁻ ; 138⁻ = [M - OCH₂Ph]⁻

### Stade c : Estérification

### 2-[[(phénylméthoxy)carbonyl]amino]-4-pentynoate de 1,1-diméthyléthyle.

A 2,6 g d'acide obtenu au stade précédent dans 65 ml de DMAC, on ajoute 25,75 g de carbonate de potassium, 1,66 g de chlorhydrate de triéthylbenzylammonium (BTEAC) et 30 ml de bromure de ter-butyle, et agite 16 heures et 45 minutes à 60°C. Après refroidissement à température ambiante, on filtre, lave, sèche et évapore sous pression réduite. On obtient 2,75 g de produit attendu.
**IR (CHCl**_{**3**}**)**
NH 3429 cm⁻¹ ; C≡CH 3309 cm⁻¹ ; C=O 1719 cm⁻¹ ; Amide II et Ph-CH₂- 1508 cm⁻¹.

### Stade d : formation du benzofurane 5-(3-méthoxy-3-oxopropyl)-.alpha.[[(phénylméthoxy)carbonyl]-amino]-2-benzofuranepropanoate de 1,1-diméthyléthyle

A 2,38 g de l'ester préparé au stade précédent, on ajoute 2,3 g du dérivé iodé P3, 800 mg de Cu₂O, 25 ml de pyridine puis porte au reflux pendant 14 heures. Après retour à température ambiante, on filtre, rince avec de l'acétate d'éthyle, évapore sous pression réduite et élimine la pyridine résiduelle en codistillant avec du toluène. On purifie par chromatographie en éluant avec le mélange dichlorométhane/acétate d'éthyle 95/5 puis 97/3 et reprend le produit avec de l'acétate d'éthyle, lave, sèche et évapore sous pression réduite jusqu'à obtention de 3,3 g de produit attendu que l'on repurifie par chromatographie en éluant avec le mélange cyclohexane/acétate d'éthyle 8/2. On obtient 2,86 g de produit pur attendu.
**RMN (CDCl**_{**3**}**)**
1,44 (s, BOC) ; 2,65(t, 2H) ; 3,01 (t, 2H) 3,29 (dl, 2H) ; 4,60 (m, 1H) ; 5,43 (d, 1H) ; 3,67 (s, 3H) ; 5,10 (AB, 2H) 6,39 (s, 1H) ; 7,05 (dd, 1H) ; 7,25 à 7,40 (m, 7H aromatique)

### Préparation 2 (P2) : Déprotection de P1

### .alpha.amino-5-(3-méthoxy-3-oxopropyl)-2-benzofuranepropanoate de 1,1-diméthyléthyle

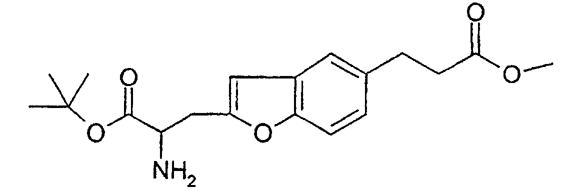

On ajoute 700 mg de palladium sur carbone à 1,05 g d'amine protégée de la préparation 1 dans 50 ml de méthanol, purge l'argon et fait passer un courant d'hydrogène, puis après filtration évapore sous pression réduite jusqu'à obtention de 626 mg de produit déprotégé attendu

### Préparation 3 (P3)

### 4-hydroxy-3-iodobenzenepropanoate de méthyle

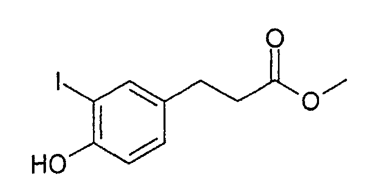

Ce composé est préparé selon les méthodes décrites par F. G. Schreiber et al. (Chem. Lett. (1975) 1257-1258 ; J. Chem. Soc. Perkin Trans (1976) 1514-1518), par B. Loev et al. (Tet. Lett (1974) 13, 1101-1103) ou encore par iodation électrophile du dérivé non iodé correspondant le 4-hydroxybenzènepropanoate de méthyle par action d'iode en présence d'hypochlorite de sodium puis de soude dans le méthanol.

### Exemple 1 : Acide 5-[3-oxo-3-[(1,4,5,6-tétrahydro-2-pyrimidinyl)amino)propyl]-.alpha.-[[(phénylméthoxy)carbonyl]amino]-2-benzofuranepropanoique

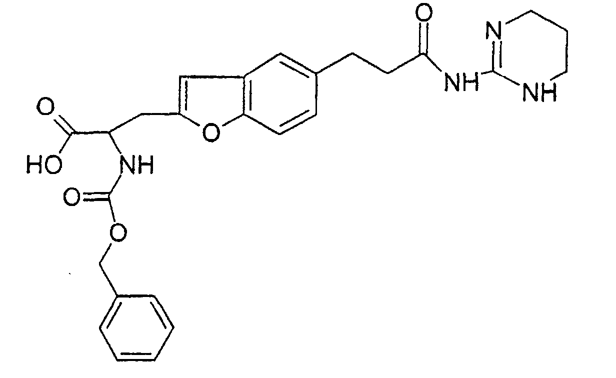

### Stade a : Formation de l'acylguanidine 5-[3-oxo-3-[(1,4,5,6-tétrahydro-2-pyrimidinyl)amino)propyl]-.alpha.-[[(phénylméthoxy)carbonyl]amino]-2-benzofuranepropanoate de 1,1-diméthyléthyle

A 1,5 g de produit obtenu à la préparation 1 (P1) dans 10 ml de THF, on ajoute 620 mg de guanidine cyclique (1,4,5,6-tétrahydro-2-pyrimidinamine) et agite 4 heures 30 minutes sous azote à température ambiante. Après évaporation sous pression réduite, on purifie par chromatographie 4,2 g de produit brut (2 essais ont été réunis) en éluant avec le mélange solvant A/acétate d'éthyle 1/1 (solvant A : CHCl₃ 70, MeOH 30, AcOH 6, H₂O 3). On obtient 1,1g de produit attendu Rf = 0,25 (solvant A/Acétate d'éthyle 1/1).

### Stade b : Hydrolyse de l'ester

### Acide 5-[3-oxo-3-[(1,4,5,6-tétrahydro-2-pyrimidinyl)amino)-propyl]-.alpha.-[[(phénylméthoxy)carbonyl]amino]-2-benzofuranepropanoique.

A 1,09 g de l'ester obtenu au stade précédent dans 12 ml de dichlorométhane, on ajoute 12 ml d'anhydride trifluoroacétique, agite 3 heures à température ambiante et évapore sous pression réduite jusqu'à obtention de 1,06 g de produit attendu que l'on purifie par chromatographie en éluant avec le mélange Solvant A/Acétate d'éthyle 1,5/0,5, pour obtenir 893 mg de produit amorphe que l'on reprend avec 10 ml de CH₂Cl₂ et 10 ml de TFA. On obtient après évaporation sous pression réduite 953 mg de produit pur attendu.
**RMN (CDCl**_{**3**}**)**
1,91 (m, CH₂ en 5') ; 2,80 (tl) 3,02 (m) =C-CH₂-CH₂-Ar ; 3,37 (m, 6H CH₂ en 4' et 6' et =C-CH₂-CH-NHCO) ; 4,75 (m,=C-CH₂-CH-NHCO) ; 5,13 (sl, -CO₂CH₂Ph) ; 5,60 (d, =C-CH₂-CH-NHCO); 7,01 (dl) 7,17 (dl) 7,24 (masqué) phényle du benzofurane ; 7,35 (sl, phényle du benzyle) ; 9,95 (sl, H mobiles)
**SM** 493⁺ = MH⁺ ; 985⁺ = 2MH⁺

### Exemple 2 : 5-[3-oxo-3-[(1,4,5,6-tétrahydro-2-pyrimidinyl) amino]propyl-.alpha.-[[(phénylméthoxy)carbonyl]amino]-2-benzofuranpropanoate de 1-méthyléthyle.

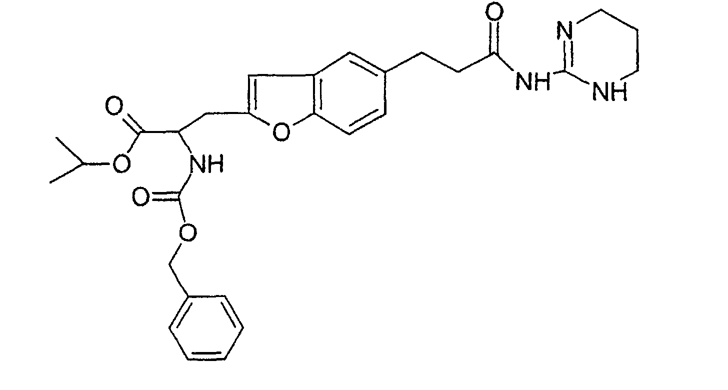

A 700 mg de d'ester terbutylique de l'exemple 1 on ajoute 25 ml d'isopropanol, 100 mg d'acide sulfurique et porte au reflux 14 heures puis ajoute encore 100 mg d'acide sulfurique et poursuit le reflux pendant 24 heures. Après évaporation sous pression réduite, on reprend avec de l'acétate d'éthyle, lave, sèche, évapore sous pression réduite, purifie par chromatographie en éluant avec le mélange solvant A/Acétate d'éthyle 1/1, et obtient 493 mg de produit que l'on reprend par le THF et 2 ml d'acide chlorhydrique. On évapore sous pression réduite en rajoutant du toluène et recristallise dans de l'éther. On obtient 476 mg de produit pur.
**RMN (CDCl**_{**3**}**)**
1,24 (m, (CH₃)₂CHOCO-) ; 5,06 (m, (CH₃)₂CHOCO-) ; 2,02 (m, CH₂ en 5') ; 2,90 (tl) 3,02 (m) =C-CH₂-CH₂-Ar ; 3,46 (m, CH₂ en 4' 6') ; 3,31 (d,=C-CH₂-CH-NHCO) ; 4,68 (m, =C-CH₂-CH-NHCO) ; 5,49 (d,=C-CH₂-CH-NHCO) ; 5,10 (AB , -CO₂CH₂Ph) ; 6,40 (s, H3) ; 7,08 à 7,40 (m, H aromatique) ; 9,41 (s, 2H) 12,81 (s, 1H) H mobiles.

### Exemple 3 : 5-[3-oxo-3-[(1,4,5,6-tétrahydro-2-pyrimidinyl) amino]propyl]-.alpha.-[[(phénylméthoxy)carbonyl]amino]-2-benzofuranpropanoate d'éthyle

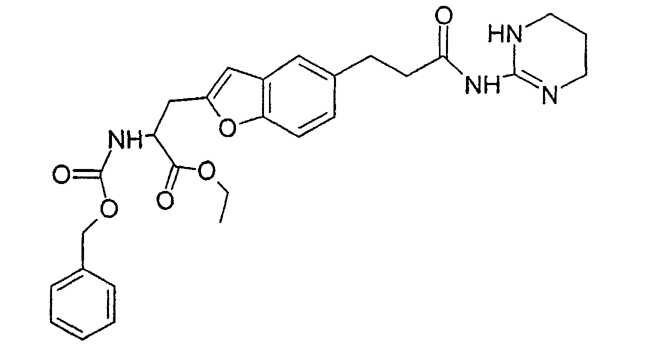

On opère comme à l'exemple 2 mais en présence de 10 ml d'éthanol à la place de l'isopropanol. On obtient 392 mg de produit attendu.
**RMN (CDCl**_{**3**}**)**
1,23 (m, CH₃CH₂OCO-) ; 4,13 (q) 4,23 (m)CH₃CH₂OCO- ; 1,93 (m, CH2 en 5') ; 2,64 (m) 3,00 (m) =C-CH₂-CH₂-Ph ; 4,48 (q) 4,71 CO-CH-NHCO ; 5,10(m CH₂O) ; 5,51(sl) 5,73(d) 5,86(d) NH- ; 6,38(s, H3) ; 7,09(dd,H6) ; 7,24 à 7,35 (H aromatiques, H4, H7) ; 7,85 H mobile.
**SM** 521⁺ = [M + H]⁺ ; 1041 ⁺ = [ 2M + H]⁺ ; 519⁻ = [M - H]⁻ ; 411⁻ = MH⁻-OCH₂Ph.

### Exemple 4 : Monochlorhydrate de 5-[3-oxo-3-[(1,4,5,6-tétrahydro-2-pyrimidinyl)amino]propyl]-.alpha.-[[(phénylméthoxy) carbonyl]-amino]-2-benzofuranpropanoate d'éthyle

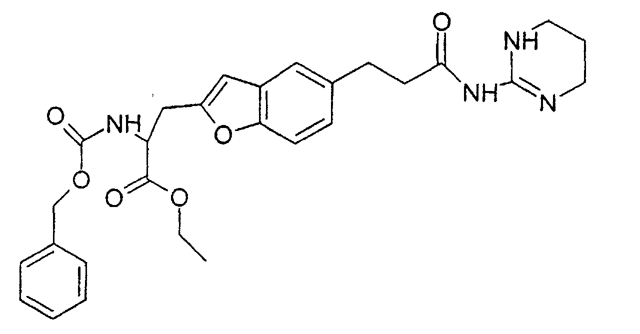

A 339 mg de l'ester éthylique de l'exemple 3 dans 20 ml de THF, on ajoute 3 ml d'acide chlorhydrique 2N agite 10 minutes et évapore sous pression réduite en rajoutant du toluène. Après traitement avec de l'éther et de l'éther isopropylique on obtient 312 mg du sel d'acide chlorhydrique attendu.

### Exemple 5 : Mono(trifluoroacétate) de l'acide 5-[3-oxo-3-[(1,4,5,6-tétrahydro-2-pyrimidinyl)amino]propyl]-.alpha.-[[[(tricyclo[3.3.1.1^{3,7}]dec-1-yl)méthoxy]carbonyl]amino]-2-benzofuranpropanoique.

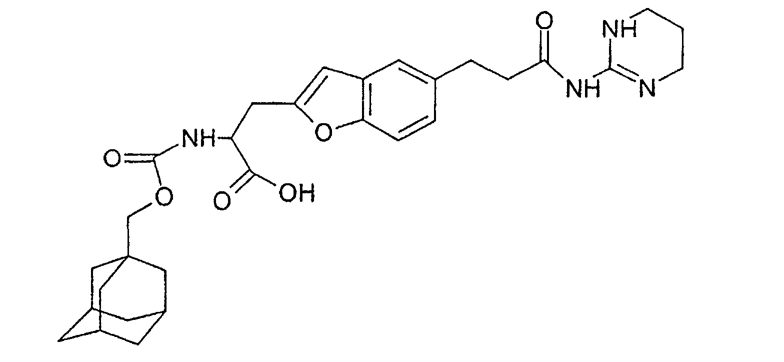

### Stade a : .alpha.-[[[(tricyclo[3.3.1.1^{3,7}]dec-1-yl)méthoxy]carbonyl]-amino]-5-(3-méthoxy-3-oxopropyl)-2-benzofuranpropanoate de 1,1-diméthyléthyle

A 150 mg d'amine P2 dans 10 ml de dichlorométhane, sous argon et à 0°C, on ajoute 78 µl de triéthyamine et 164 mg de 1-[[[(tricyclo[3.3.1.1^{3,7}]dec-1-yl)méthoxy]carbonyl]oxy]-2,5-pyrrolidinedione (cf J. Chem. Soc. Chem. Comm. (1992) 1308) et agite 18 heures en laissant la température évoluer à température ambiante. Après lavage et séchage, on évapore sous pression réduite jusqu'à obtention de 288 mg de produit attendu.
**IR (CHCl**_{**3**}**)**
NH 3434 cm⁻¹, C=O 1743 cm⁻¹ (Max), Système conjugué + amide II : 1600, 1507 cm⁻¹.

### Stade b : Amidification

### 5-[3-oxo-3-[(1,4,5,6-tétrahydro-2-pyrimidinyl)amino]propyl]-.alpha.-[[[(tricyclo[3.3.1.1^{3.7}]dec-1-yl)méthoxy]carbonyl]-amino]-2-benzofuranpropanoate de 1,1-diméthyléthyle.

On opère comme à l'exemple 1 à partir de 220 mg d'ester préparé au stade a et 80 mg de 1,4,5,6-tétrahydro-2-pyrimidinamine. On obtient 120 mg de produit attendu.
IR (CHCl₃)
NH 3433 ; 3265 cm⁻¹, C=O complexe 1712 cm⁻¹ (Max ; C=O C=N 1658 cm⁻¹ ; Aromatique + C=C + Amide II : 1601, 1574, 1508 cm⁻¹.

### Stade c : hydrolyse de l'ester terbutylique

### Mono(trifluoroacétate) de l'acide 5-[3-oxo-3-[(1,4,5,6-tétrahydro-2-pyrimidinyl)amino]propyl]-.alpha.-[[[(tricyclo-[3.3.1.1^{3,7}]dec-1-yl)méthoxy]carbonyl]amino]-2-benzofuranpropanoique

On ajoute 2 ml d'acide trifluoroacétique à une solution de 120 mg d'ester préparé au stade précédent dans 1 ml de dichlorométhane et agite 4 heures à température ambiante. On évapore sous pression réduite, reprend avec un mélange eau/acide acétique et lyophilise pour obtenir 65 mg de produit attendu
**SM :** 551 Da = MH⁺

### Exemple 6 : Acide 5-[3-oxo-3-[(1,4,5,6-tétrahydro-2- pyrimidinyl)amino]-propyl]-.alpha.-[[[[[(tricyclo [2.3.1.1^{3,7}]dec-1-yl)méthoxy]-carbonyl]amino]sulfonyl]amino]-2-benzofuranpropanoique

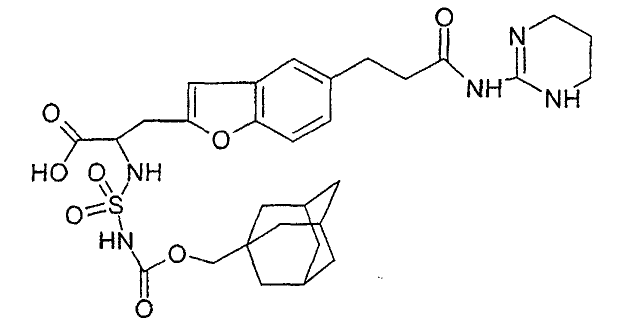

### Stade a : Addition du chlorosulfonyle 5-(3-méthoxy-3-oxopropyl).alpha.-[[[[[(tricyclo[2.3.1.1^{3,7}]-dec-1-yl)méthoxy]carbonyl]amino] sulfonyl]amino] -2-benzofuranpropanoate de 1,1-diméthyléthyle.

A une solution de 250 mg d'isocyanate de chlorosulfonyle dans 5 ml de dichlorométhane, on ajoute 292 mg de tricyclo-[3.3.1.13,7]decane-1-méthanol dans 5 ml de dichlorométhane et agite à 0°C pendant 1 heure trente minutes. On ajoute ensuite 692 mg d'amine P2 puis 490 µl de TEA. Après lavage par une solution d'acide chlorhydrique 1N/NaHCO₃ puis séchage, on évapore sous pression réduite et purifie par filtration en lavant avec du dichlorométhane. On obtient après purification 0,88 g de produit attendu.
**IR (CHCl**_{**3**}**)**
NH 3391 cm⁻¹ ; C=O 1736 cm⁻¹ ; Système conjugué + aromatique 1600, 1473 cm⁻¹.

### Stade b : Amidification

### (3.alpha.,5.beta.,7.alpha.)-5-[3-oxo-3-[(1,4,5,6-tétrahydro-2-pyrimidinyl)amino]propyl]-.alpha.-[[[[[(tricyclo-[2.3.1.1^{3,7}]dec-1-yl)méthoxy]carbonyl]amino]sulfonyl]amino]-2-benzofuranpropanoate de 1,1-diméthyléthyle.

On opère comme à l'exemple 1 mais à partir de 0,88 g de l'ester préparé au stade précédent et 280 mg de (1,4,5,6-tetrahydro-2-pyrimidinamine). On obtient 220 mg de produit attendu.
**RMN (CDCl**_{**3**}**)**
1,43 (s, BOC) ; 1,50 à 2,05 (Adamantyle) ; 1,95 (CH₂ en 5') 2,83 (t) 3,01 (t) Ar-CH₂-CH₂-CO ; 3,27 (m, CO₂CH₂-Adam)); 3,41 (t, 4H,CH₂ en 4' 6') ; 3,67 (AB, =C-CH₂-CH) ; 4,47 (t, SO₂NHCHCH₂-C=) ; 6,47 (s, H3) ; 7,07 (dd, H6) ; 7,24 (d, H7) ; 7,33 (sl, H4).

### Stade c : Hydrolyse

### Acide 5-[3-oxo-3-[(1,4,5,6-tétrahydro-2-pyrimidinyl)amino] propyl]-.alpha.-[[[[[(tricyclo-[2.3.1.1^{3,7}]dec-1-yl)méthoxy] carbonyl]amino]sulfonyl]amino] -2-benzofuranpropanoique

On opère comme à l'exemple 5 stade c à partir de 220 mg de l'ester ter butylique obtenu au stade précédent et 1,5 ml d'anhydride trifluoroacétique. On obtient 210 mg de produit attendu
**RMN (DMSO d**_{**6**}**)**
1,43 à 1,91 (Adamantyle) ; 2,77 (t, J=7,5) 2,96 (t, J=7,5) Ar-CH₂-CH₂-CO; 3,39 (d) 3,47 (d, J_{AB}=10,5) CO₂CH₂-C ; 1,86 (m, CH2 5') ; 3,36 (1, 4H, CH₂ 4' 6') ; 9,32 (s, NH) ;6,58 (s, H3); 7,40 (d, J=1,5 H4) ; 7,11 (dd, J=1,5-8,5 H6) ; 7,38 (d, J=8,5 H7) ; 8,24 (d, J=8,5 NH) ; 4,31 (td, J=8,5-15,5 SO₂NHCHCH₂-C=) ; 3,09 (dd,J=8,5-15,5) 3,20 (dd, J=8,5-15,5) SO₂NHCHCH₂-C=.

### Exemple 7 : Acide 5-[3-oxo-3-[(1,4,5,6-tétrahydro-2-pyrimidinyl)amino]-propyl]-.alpha.-[[[[(phényl)méthoxy] carbonyl]-amino]-sulfonyl]amino]-2-benzofuranpropanoique

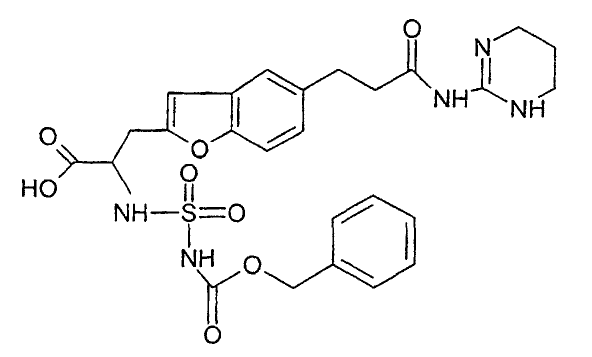

On opère comme à l'exemple 6 stades a, b et c mais, en ce qui concerne le stade a, à partir d'alcool benzylique à la place de tricyclo[3.3.1.1^{3,7}]decane-1-méthanol.
**RMN (CDCl**_{**3**}**)**
1,84 (m, CH₂ en 5') ; 2,75 (t) 2,94 (t, Ar-CH₂-CH₂-C=O) ; 3,07 (dd) 3,20 (dd) =C-CH₂-CH-NHSO₂ ; 4,31 (m, =C-CH₂-CH-NHSO₂) ; 4,87 (s, CO₂CH₂Ph) ; 6,59 (s, H3) ; 7,10 (dd, H6) ; 8,43 (d, H7) ; 7,29 à 7,43 (Benzyle, H4) ; 9,19 (sl, 2H) 11,30 (s, 1H) 11,82 (s, 1H) H mobiles.

### Exemple 8 : Mono(trifluoroacétate) de l'acide.alpha.-[[[(phénylméthyl)-amino]sulfonyl]amino]-5-[3-oxo-3-[(1,4,5,6-tétrahydro-2-pyrimidinyl)amino]propyl]-2-benzofuranpropanoique

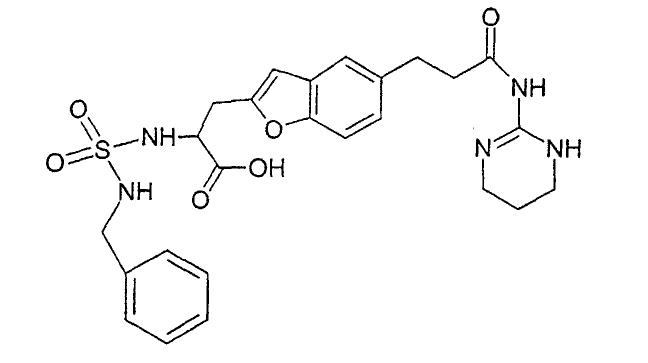

### Stade a : Addition du chlorosulfonyle

### .alpha.-[[[[(1,1-diméthyléthoxy)carbonyl]amino]sulfonyl]-amino]-5-(3-méthoxy-3-oxopropyl)-2-benzofuranpropanoate de 1,1-diméthyléthyle.

A une solution de 158 µl d'isocyanate de chlorosulfonyle dans 5 ml de dichlorométhane, on ajoute 131 mg d'alcool terbuty-lique dans 5 ml de dichlorométhane et agite à 0°C pendant 1 heure trente minutes. On ajoute ensuite 692 mg d'amine P2 puis 490 µl de TEA. Après lavage puis séchage, on évapore sous pression réduite et purifie par chromatographie en éluant avec le mélange dichlorométhane/acétone 95/5. On obtient après purification 630 mg de produit attendu
**IR (CHCl**_{**3**}**)**
NH 3395 cm⁻¹ ; C=O 1736 cm⁻¹ ; Système conjugué + aromatique 1600 cm⁻¹.

### Stade b : Alkylation de l'amine

### alpha.-[[[[(1,1-diméthyléthoxy)carbonyl](phénylméthyl)-amino]sulfonyl]amino]-5-(3-méthoxy-3-oxopropyl)-2-benzofuranpropanoate de 1,1-diméthyléthyle

On mélange à température ambiante pendant 30 minutes 630 mg de la sulfonylurée obtenue au stade précédent dans 8 ml de dichlorométhane avec 185 µl d'alcool benzylique et 736 mg de bétaine : ((T-4)-[(3,3-diméthyl)-1,2,5-thiadiazolidinekappa.N5)1,1-dioxidato(2-)]triphényl-phosphorus), préparé selon J. Org. Chem (1994) 59, 2289 puis après lavage, séchage et évaporation sous pression réduite purifie par chromatographie en éluant avec le mélange dichlorométhane/acétone 95/5. On obtient 310 mg de produit attendu
**IR (CHCl**_{**3**}**)**
NH 3320 cm⁻¹ ; C=O 1732 cm⁻¹ ; C=C + aromatique 1602, 1496 cm⁻¹.

### Stade c : Formation de l'acylguanidine

### .alpha.-[[[[(1,1-diméthyléthoxy)carbonyl] (phénylméthyl)-amino]sulfonyl]amino]-5-[3-oxo-3-[(1,4,5,6-tétrahydro-2-pyrimidinyl)amino]propyl]-2-benzofuranpropanoate de 1,1-diméthyléthyle

On opère comme à l'exemple 1 mais à partir de l'ester préparé au stade précédent et 4 équivalents de 1,4,5,6-tétrahydro-2-pyrimidinamine. On obtient 220 mg de produit attendu.
**RMN (CDCl**_{**3**}**)**
1,39 (s) 1,47 (s) 2 BOC ; 1,95 (m, CH₂ en 5') ; 3,40 (m, CH₂ en 4' et 6') ; 2,85 (t) 3,01 (t) Ar-CH₂-CH₂-CO ; 3,13 (m, =C-CH₂-CH-NHCO) ; 3,81 (m, =C-CH₂-CH-NHCO) ; 6,04 (m, =C-CH₂-CH-NHCO) ; 4,73 (d, J=15,5) 4,94 (d, J=15,5) X-CH₂-Ph ; 6,43 (s, H3) ; 7,10 (dd, H6) ; 7,20 à 7,40 (m, 7H aromatique).

### Stade d : Hydrolyse puis déprotection

### Mono(trifluoroacétate) de l'acide.alpha.-[[[(phénylméthyl)-amino]sulfonyl]amino]-5-[3-oxo-3-[(1,4,5,6-tétrahydro-2-pyrimidinyl)amino]propyl]-2-benzofuranpropanoique.

On opère comme à l'exemple 5 stade c à partir de 306 mg de l'ester tert-butylique obtenu au stade précédent et 2 ml d'acide trifluoroacétique. On obtient 250 mg de produit attendu.
**RMN (DMSO d**_{**6**}**)**
1,84 (m, CH₂ en 5') ; 3,34 (m, CH₂ en 4' et 6'), 2,77 (t) 2,95 (t) Ar-CH₂CH₂-CO ; 3,04 (dd) 3,17 (dd) =C-CH₂-CH-NHSO₂ ; 4,12 (m, =C-CH₂-CH-NHSO₂) ; 7,59 (d, =C-CH₂-CH-NHSO₂) ; 3,67 (dd) 3,83 (dd) Ph-CH₂-NH ; 7,40 (masqué, Ph-CH₂-NH) ; 6,64 (s, H3) ; 7,11 (m, 3H aromatique) ; 7,23 (m, 3H aromatique) ; 7,40 (m, 2H aromatique) ; 8,98 (s, 2H mobiles) ; 11,53 (s, 1H mobiles) ; 12,90 (large, 1H mobile)

### Exemple 9 : Acide .alpha.-[[[4-(1,1-diméthyléthyl)phényl] sulfonyl]amino]-5-(3-oxo-3-[(1,4,5,6-tétrahydro-2-pyrimidinyl)amino]propyl]-2-benzofuranpropanoique

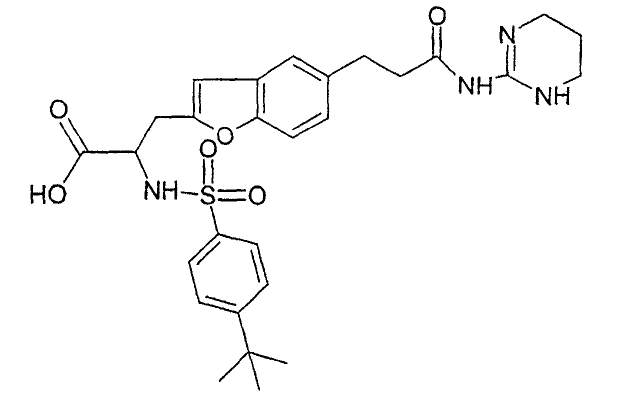

### Stade a : .alpha.-[[4-(1,1-diméthyléthyl)phénylsulfonyl] amino]-5-(3-méthoxy-3-oxopropyl)-2-benzofuranpropanoate de 1,1-diméthyléthyle.

A 347 mg de P2 dans 3 ml de pyridine, on ajoute sous azote 24 mg de 4-diméthylaminopyridine et 466 mg de chlorure de 4-tert-butyl-benzosulfonyle et agite 16 heures au reflux. Après refroidissement, on verse ensuite dans 10 ml d'eau extrait avec du dichiorométhane, lave, sèche et évapore sous pression réduite jusqu'à obtention de 590 mg que l'on purifie par chromatographie en éluant avec le mélange cyclohexane/acétate d'éthyle 7/3. On obtient 500 mg de produit pur attendu. Rf cyclohexane/acétate d'éthyle 7/3 = 0,31.
**IR (CHCl**_{**3**}**)** NH 3340 cm⁻¹ ; C=O 1733 cm⁻¹ ; C=C aromatique 1597 cm⁻¹ ; SO₂ 1348, 1156 cm⁻¹.

### Stade b : Formation de l'acyle guanidine

### .alpha.-[[(4-(1,1-diméthyléthyl)phénylsulfonyl]amino]-5-(3-oxo-3-[(1,4,5,6-tétrahydro-2-pyrimidinyl)amino]propyl]-2-benzofuranpropanoate de 1,1-diméthyléthyle

On mélange pendant 3 heures à température ambiante 500 mg de l'ester préparé au stade précédent dans 4 ml de THF et 200 mg de 1,4,5,6-tétrahydro-2-pyrimidinamine Puis on évapore sous pression réduite pour obtenir 600 mg de produit brut que l'on purifie par chromatographie en éluant avec le mélange solvant A/acétate d'éthyle 1/1. On obtient 312 mg de produit purifié attendu.
**IR (CHCl**_{**3**}**)**
NH complexe 3269 cm⁻¹ ; C=O 1731, 1689 cm⁻¹ ; système conjugué + aromatique 1663, 1597, 1576, 1496 cm⁻¹ ; SO₂ 1349, 1155 cm⁻¹.

### Stade c : hydrolyse

### Acide .alpha. - [[[4-(1,1-diméthyléthyl)phényl]sulfonyl]amino]-5-(3-oxo-3-[(1,4,5,6-tetrahydro-2-pyrimidinyl)amino]propyl]-2-benzofuranpropanoique.

On ajoute 3 ml d'anhydride trifluoroacétique à 305 mg d'acylguanidine préparé au stade précédent dans 3 ml de dichlorométhane, agite à température ambiante pendant 3 heures puis évapore sous pression réduite, en rajoutant du toluène, jusqu'à obtention de 300 mg de produit brut sous la forme d'un sel d'acide trifluoroacétique. On purifie par chromatographie en éluant avec le solvant A/AcOEt 1,5 : 0,5. Le résidu obtenu est repris dans 10 ml d'eau, on mélange CH₂Cl₂/TFA : 1/1 puis on évapore sous pression réduite, reprend dans du toluène et reconcentre à sec. On obtient 254 mg de produit attendu.
**RMN (CDCl**_{**3**}**)**
1,29 (s, Ph-tBu) ; 1,88 (m, CH₂ en 5') ; 3,33 (m, CH₂ en 4' et 6'), 2,76 (m) 2,98 (m) Ar-CH₂CH₂-CO ; 3,21 (dd, =C-CH₂-CH-NH) ; 4,30 (m, =C-CH₂-CH-NH) ; 5,20 (m, 2H mobile) ; 5,73 (m, 1H mobile) ; 6,37 (sl, H3) ; 6,99 (dl) 7,20 (m), H4 H6 H7 ; 7,39 et 7,70 (AA'BB', phényle) ; 9,82 (s large, 2H mobiles) ; 12,64 (s, 1H mobile)

### Exemple 10 : Acide .alpha.-[[[4-(1-méthyléthyl)phényl] sulfonyl]amino]-5-(3-oxo-3-[(1,4,5,6-tétrahydro-2-pyrimidinyl)amino]propyl]-2-benzofuranpropanoique

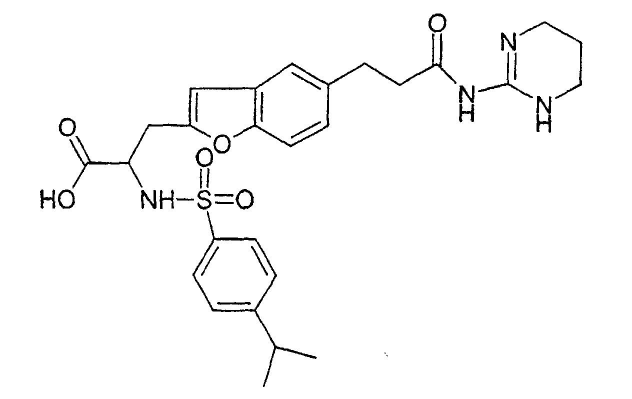

On opère comme à l'exemple 9 mais à partir de chlorure de 4-isopropyl-benzosulfonyle.
**RMN (CDCl**_{**3**}**)**
1,22 (d, (CH₃)₂CH) 2,90 (m, (CH₃)₂CH) ; (s, Ph-tBu) ; 1,88 (m, CH₂ en 5') ; 3,33 (m, CH₂ en 4' et 6'), 2,77 2,98 Ar-CH₂CH₂-CO ; 3,19 (=C-CH₂-CH-NHSO₂) ; 6,37 (s, H3) ; 6,99 (d, 1H benzofurane) ; 7,18 (2H, benzofurane) ; 7,21 et 7,68 (phényle) ; 9,84 (NH₂⁺ couplé à 3,33) ; 12,67 (s, 1H mobile).

### Exemple 11 : Acide 5-[3-oxo-3-[(1,4,5,6-tétrahydro-2-pyrimidinyl)amino]-propyl]-.alpha.-[propylsulfonyl)amino]-2-benzofuran-propanoique.

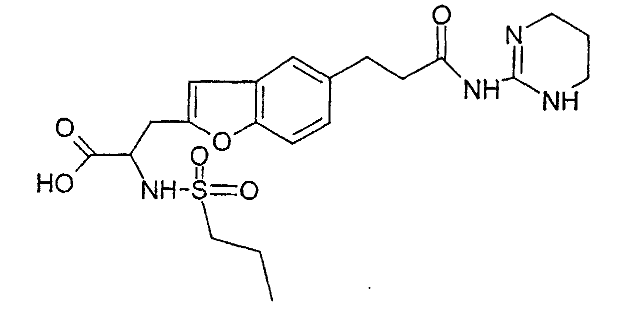

On opère comme à l'exemple 9 mais à partir de chlorure de 4-n-propyl-benzosulfonyle.
**RMN (DMSO d**_{**6**}**)**
0,67 (t) 1,47 (m) 2,75 (m) CH₃-CH₂-CH₂-SO₂ ; 1,85 (m, CH₂ en 5') ; 2,50 (masqué, =N-CH₂-CH₂) ; 3,35 (m, CH₂ en 4' et 6'), 2,75, 2,95(m, 4H Ar-CH₂CH₂-CO) ; 4,23 (m, =C-CH₂-CH-NHSO₂) ; 6,64 (s, H3) ; 7,12 (d, 1H H7) ; 7,42 (m, 2H, H4 et H6) ; 9,29 (sl, 2H mobiles) ; 11,96 (s, 1H mobile) ; 13,14 (1, 1H mobile).

### Exemple 12 : Acide.alpha.-[méthylsulfonyl)amino]-5-[3-oxo-3-[(1,4,5,6-tétrahydro-2-pyrimidinyl) amino]propyl]--2-benzofuranpropanoique.

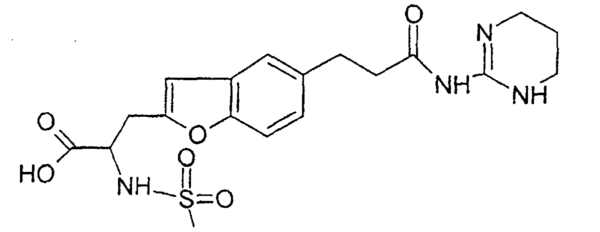

On opère comme à l'exemple 9 mais à partir de chlorure de 4-méthyl-benzosulfonyle.
**RMN (CDCl**_{**3**}**)**
1,85 (m, CH₂ en 5') ; 2,50 (masqué, =N-CH₂-CH₂) ; 3,35 (m, CH₂ en 4' et 6'), 2,75 (t) 2,95 (t) Ar-CH₂CH₂-CO ; 2,90 (m, 2H, SO₂CH₃) ; 3,09 (dd) 3,24 (dd) =C-CH₂-CH-NHSO₂ ; 4,28 (m, =C-CH₂-CH-NHSO₂) ; 7,79 (d, =C-CH₂-CH-NHSO₂) 6,64 (s, H3) ; 7,12 (d, 1H H7) ; 7,42 (m, 2H, H4 et H6) ; 9,30 (s) 11,96 (s) 13,14 (1), H mobiles.

### Exemple 13 : Acide. alpha.-[(1-naphtalenylsulfonyl)amino]-5-[3-oxo-3-[(1,4,5,6-tétrahydro-2-pyrimidinyl)amino]propyl]--2-benzofuranpropanoique.

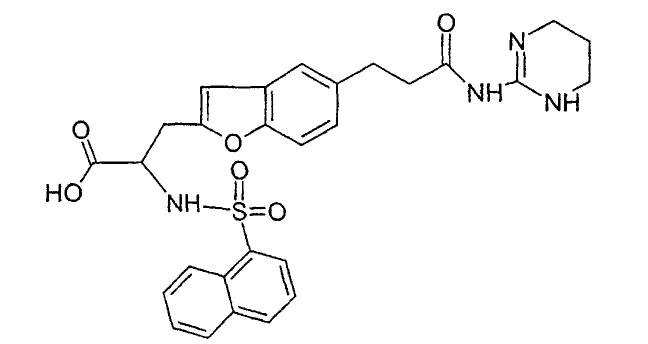

On opère comme à l'exemple 9 mais à partir de chlorure de sulfonyle-1-naphtalène

### Exemple 14 : alpha.-[(1-naphtalenylsulfonyl)amino]-5-[3-oxo-3-[(1,4,5,6-tétrahydro-2-pyrimidinyl)amino]propyl]--2-benzofuranpropanoate de 1-méthyléthyle.

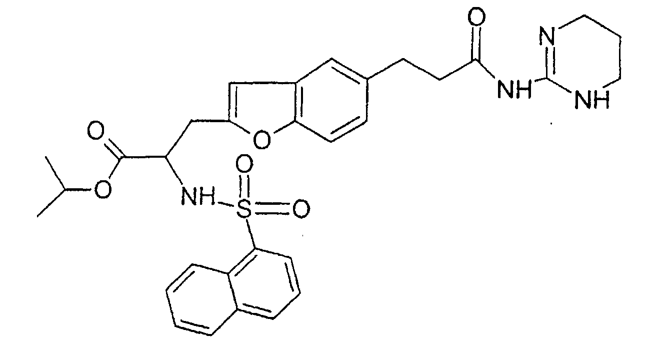

On opère comme à l'exemple 2 à partir de 648 mg d'acide préparé à l'exemple 13 et 20 ml d'isopropanol. On obtient 350 mg de produit attendu.
**RMN (CDCl**_{**3**}**)**
0,98 (d, (CH₃)₂CH) ; 4,77 (m, (CH₃)₂CH) ; 2,02 (m, CH₂ en 5') ; 3,48 (m, CH₂ en 4' et 6'), 2,89 (t) 3,06 (t) Ar-CH₂CH₂-CO ; 3,05 (dd) 3,15 (dd) =C-CH₂-CH-NHSO2 ; 4,28 (m, =C-CH₂-CH-NHSO2) ; 5,70 (d, =C-CH₂-CH-NHSO₂) 6,35 (s, H3) ; 7,0 à 7,20 (3H, benzofurane) ; 7,44 (t, H3 du naphtalène) ; 8,00 (d) et 8,21 (d) H2 et H4 du naphtalène ; 7,57 (m, H6 et H7 du naphtalène) ; 7,87 8,56 (H5 et H8 du naphtalène) ; 9,40 (sl, 2H mobile) 12,84 (s, 1H mobile)

### Test pharmacologique

### Test ELISA Kistrine/Récepteur Vitronectine (α_{V}β₃) Protocole

Des plaques 96 puits MaxiSorp sont coatées une nuit à 40°C avec 100 µl de Kistrine à 1 µg/ml (dilution en tampon de coating : carbonate 0,05 M / NaOH pH 9,6). Le lendemain, les puits sont vidés et les ligands (kistrine) sont ensuite fixés (tampons de fixation : PBS contenant 0,5% de BSA (pH=7,4)) pendant 1 heure à température ambiante avec une agitation douce de 125 rpm. Les puits sont lavés six fois (tampon de lavage :PBS contenant 0,05 % de Tween 20 (pH 7,7) puis on ajoute par puits et dans cet ordre :
- 40 µl de tampon d'incubation
- 10 µl de la dilution du produit à tester (les produits sont dilués dans un mélange 50:50 DMSO/Eau)
- 50 µl de récepteur αᵥβ₃ humain (cf Pytel et al. Méthods Enzymol. (1987) 144 (Dilution en tampon d'incubation, à adapter suivant le lot de récepteur et selon le ligand). Le ligand, le récepteur α_{V}β₃ et les produits à étudier sont incubés pendant 3 heures à température ambiante avec une agitation douce de 125 rpm.

Les puits sont à nouveau lavés six fois, puis incubés pendant 2 heures à température ambiante avec une agitation douce de 125 rpm, en présence de 100 µl d'anticorps anti-récepteur couplé à une peroxydase (L'anticorps 4B12-HRP est dilué en tampon d'incubation (50 mM TRIS pH 7,4 ; 0,5 % BSA ; 0,05 % Tween 20 ; 1 mM MnCl₂ ; 50 µM CaCl₂ ; 50 µM MgCl₂ ; 100 mM NaCl). La dilution est à adapter suivant le lot de récepteur).

Les puits sont ensuite lavés six fois avant la mesure de la liaison ligand-récepteur faite par l'intermédiaire d'un kit révélateur de peroxydase (TBM Microwell Peroxidase Substrate System Kirkegaard ; Ref cat 50-76-00).

Ce kit contient un flacon A de substrat (3,3',5,5'-tétraméthylbenzidine à 0,4 g/l) et un flacon B (H₂O₂ à 0,02 % en tampon Citrate/Acide citrique). Extemporanément, un volume de A est mélangé à un volume de B, puis le mélange réactionnel est distribué à raison de 100 µl/puits.

La réaction enzymatique se développe entre 6 à 10' pour Kistrine/α_{V}β₃ puis son évolution est stoppée par l'addition de 100 µl d'acide phosphorique 1M. La densité optique est déterminée à 450 nm.

### Expression des résultats

On trace la courbe suivante : le pourcentage de liaison en fonction du logarithme de chaque concentration du produit testé. Pour chaque produit, on détermine l'IC50 suivant la formule suivante : IC50=(B0+ Bmin)/2
B0 = Maximum de liaison en l'absence de tout produit
Bmin = Minimum de liaison en présence de la concentration la plus élevée de produit.

| **Exemple** | **K/VnR IC50 (µM)** |
|---|---|
| 1 | 0,009 |
| 5 | 0,045 |
| 7 | 0,01 |
| 6 | 0,065 |
| 8 | 0,0087 |
| 13 | 0,0055 |
| 14 | 3,7 |
| 9 | 0,015 |
| 10 | 0,0091 |
| 11 | 0,0057 |
| 12 | 0,0067 |

## Revendications

1. Un composé de formule (I) 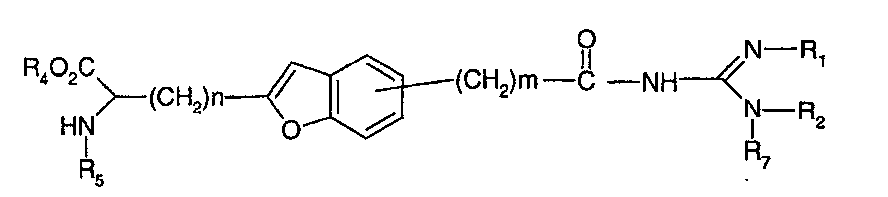 dans lequel
soit R₁ et R₂, indépendamment l'un de l'autre, représentent un atome d'hydrogène ou un groupement alkyle renfermant de 1 à 6 atomes de carbone non substitué ou substitué par R₃,
soit R₁ et R₂ forment ensemble un radical alkylène bivalent renfermant de 2 à 9 atomes de carbone, saturé ou insaturé, non substitué ou substitué par un ou plusieurs radicaux choisis parmi halogène, (C₁-C₆)-alkyle, (C₁-C₆)-alkoxy, (C₆-C₁₄)-aryle, (C₆-C₁₄)-aryle- (C₁-C₆)-alkyle, (C₅-C₁₄)-hétéroaryle, (C₅-C₁₄)-hétéroaryle-(C₁-C₆)-alkyle, (C₃-C₁₂)-cycloalkyle, (C₃-C₁₂)-cycloalkyle-(C₁-C₆)-alkyle et oxo, ledit radical alkylène bivalent pouvant être accolé au niveau de la liaison carbone-carbone à un carbocycle ou un hétérocycle de 5 à 7 chaînons, renfermant 1 ou 2 atomes d'azote, saturé ou insaturé, non substitué ou substitué par 1 ou 2 radicaux R₃ ;
R₃ représente un groupement (C₁-C₈)-alkyle, (C₁-C₈)-alkoxy, (C₆-C₁₄)-aryle, (C₆-C₁₄)-aryle-(C₁-C₄)-alkyle, (C₅-C₁₄)-hétéroaryle, (C₅-C₁₄)-hétéroaryle-(C₁-C₄)-alkyle, halogène, trifluorométhyle, hydroxyle, nitro, amino, NH-((C₁-C₄)-alkyle), N((C₁-C₄)alkyle)₂, NHCO-(C₁-C₄)-alkyle ou CO-(C₁-C₄)alkyle ;
R₄ représente
soit un atome d'hydrogène, soit un groupement (C₁-C₆)-alkyle-CO-O-(C₁-C₄)-alkyle ou (C₁-C₆)-alkyle, non substitué ou substitué par un radical choisi parmi hydroxyle, (C₁-C₄)-alkoxy, (C₁-C₄)-alkyle-SO₂, NR₉R₉' et N⁺R₉R₉'R₉"Q⁻, dans lesquels R₉, R₉' et R₉" indépendants les uns des autres, représentent un hydrogène, un groupement (C₁-C₆)-alkyle, (C₆-C₁₄)-aryle, (C₆-C₁₄)-aryle-(C₁-C₄)-alkyle , (C₅-C₁₄)-hétéroaryle, (C₅-C₁₄)-hétéroaryle-(C₁-C₄)-alkyle et Q⁻ est un anion physiologiquement acceptable,
soit un des radicaux suivants : 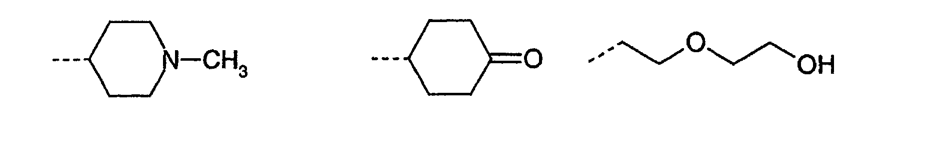 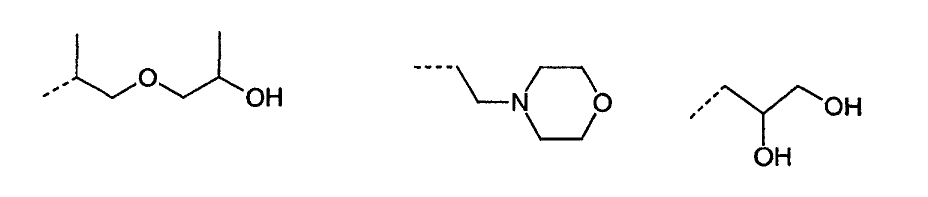 les traits en pointillés représentant la position de la liaison ;
R₅ représente un atome d'hydrogène ou un groupement choisi parmi COR₆, CO₂R₆, SO₂R₆, SO₂NHR₆, SO₂NHCOR₆, SO₂NHCO₂R₆, CONH₂ et CONHR₆ dans lesquels R₆ représente (C₁-C₈)-alkyle, (C₆-C₁₄)-aryle, (C₆-C₁₄)-aryle-(C₁-C₆)-alkyle, (C₅-C₁₄)-hétéroaryle ou (C₅-C₁₄)-hétéroaryle-(C₁-C₆)-alkyle, (C₃-C₂₀)(mono-, bi- ou tri-)-cycloalkyle, (C₃-C₂₀)(mono-, bi- ou tri-)-cycloalkyle-(C₁-C₆)alkyle, le radical aryle ou hétéroaryle étant non substitué ou substitué par 1, 2 ou 3 radicaux R₃;
R₇ représente un atome d'hydrogène, (C₁-C₆)-alkyle-O-CO-, hydroxyle, (C₁-C₆)-alkyle-O-CO-O- ou nitro ;
m est égal à 0, 1, 2 ou 3 ;
n est un entier égal à 1, 2 ou 3 ;
lesdits radicaux alkyles pouvant être mono-ou poly-insaturés, lesdits radicaux cycloalkyles pouvant être mono, bi ou tricycliques non substitués ou substitués par un ou plusieurs (C₁-C₄)-alkyles ou pour les bi- et tricycloalkyles par un ou plusieurs oxos, lesdits radicaux aryles ou hétéroaryles pouvant être non substitués ou substitués par un ou plusieurs radicaux identiques ou différents choisis parmi (C₁-C₈)-alkyle, (C₁-C₈)-alkoxy, halogène, nitro, amino, trifluorométhyle, hydroxyle, méthylènedioxy, cyano, hydroxycarbonyle, aminocarbonyle, (C₁-C₄)-alkoxycarbonyle, phényle, phénoxy, benzyle et benzyloxy, lesdits composés de formule (I) étant sous toutes leurs formes stéréo isomères possibles, seuls ou en mélange dans un rapport quelconque, le groupement acylguanidine adjacent au phényle étant en position para ou méta de l'oxygène, ainsi que leurs sels physiologiquement acceptables et leurs promédicaments (prodrugs).

2. Un composé de formule (I) tel que défini à la revendication 1 répondant à la formule (I') 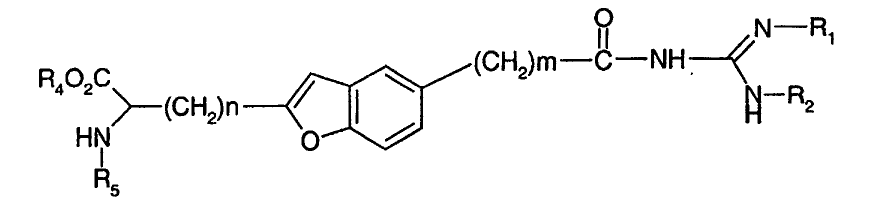 dans laquelle, R₁ et R₂ représentent un atome d'hydrogène ou forment ensemble un radical bivalent alkylène saturé ou insaturé renfermant de 2 à 5 atomes de carbone, ledit radical alkylène étant non substitué ou substitué par un ou deux radicaux identiques ou différents choisi parmi halogène, (C₁-C₆)-alkyle, (C₁-C₆)-alkoxy, (C₆-C₁₄)-aryle, (C₆-C₁₄)-aryle-(C₁-C₆)alkyle, (C₅-C₁₄)-hétéroaryle, (C₅-C₁₄)-hétéroaryle-(C₁-C₆)-alkyle, (C₃-C₁₂)-cycloalkyle et (C₃-C₁₂)-cycloalkyle-(C₁-C₆)-alkyle et oxo, ledit alkylène pouvant être accolé au niveau de la liaison carbone-carbone à un carbocycle ou un hétérocycle renfermant 1 ou 2 atomes d'azote, de 5 à 7 chaînons, saturé ou insaturé, non substitué ou substitué par R₃ ;
R₃ représente un groupement alkyle ou alkyloxy renfermant de 1 à 6 atomes de carbone ; R₄ représente un atome d'hydrogène ou un groupement alkyle renfermant de 1 à 6 atomes de carbone non substitué ou substitué par un groupe choisi parmi (C₁-C₄)-alkoxy, (C₁-C₄)-alkyle-S(O)₂ et NR₉R₉' dans lequel R₉ et R₉' indépendamment l'un de l'autre représentent un atome d'hydrogène ou (C₁-C₄)-alkyle ;
R₅ représente un atome d'hydrogène, un groupement CO₂R₆, SO₂R₆, SO₂NHR₆ ou SO₂NHCO₂R₆ dans lesquels R₆ est un radical (C₁-C₈)-alkyle ou naphtyle, non substitué ou substitué par R₃, un radical cycloalkyle renfermant de 3 à 12 atomes de carbone ou (C₃-C₁₂)-cycloalkyle-(C₁-C₆)alkyle ou le radical de formule (II) 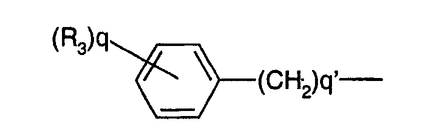 dans lequel les radicaux R₃ peuvent être identiques ou différents, et peuvent être localisés à n'importe quelle position du radical phényle, q et q' sont égaux à 0 ou 1 ;
m est un entier égal à 1, 2 ou 3 ;
n est un entier égal à 1, 2 ou 3 ;
lesdits radicaux alkyles pouvant être mono-ou poly-insaturés, lesdits radicaux cycloalkyles pouvant être mono, bi ou tricycliques non substitués ou substitués par un ou plusieurs (C₁-C₄)-alkyles ou pour les bi- et tricycloalkyles par un ou plusieurs oxos, lesdits radicaux aryles ou hétéroaryles pouvant être non substitués ou substitués par un ou plusieurs radicaux identiques ou différents choisis parmi (C₁-C₈)-alkyle, (C₁-C₈)-alkoxy, halogène, nitro, amino, trifluorométhyle, hydroxyle, méthylènedioxy, cyano, hydroxycarbonyle, aminocarbonyle, (C₁-C₄)-alkoxycarbonyle, phényle, phénoxy, benzyle et benzyloxy, lesdits composés de formule (I') étant sous toutes leurs formes stéréo isomères possibles, seuls ou en mélange selon un rapport quelconque, ainsi que leurs sels physiologiquement acceptables et leurs prodrugs.

3. Un composé de formule (I') telle que définie à la revendication 2 dans laquelle, dans laquelle, R₁ et R₂ représentent un atome d'hydrogène ou forment ensemble un radical bivalent alkylène saturé ou insaturé renfermant de 2 à 4 atomes de carbone, ledit radical alkylène étant non substitué ou substitué par un ou deux radicaux identiques ou différents choisi parmi halogène, (C₁-C₆)-alkyle, (C₁-C₆)-alkoxy, (C₆-C₁₄)-aryle, (C₆-C₁₄)-aryle-(C₁-C₆)alkyle, (C₅-C₁₄)-hétéroaryle, (C₅-C₁₄)-hétéroaryle-(C₁-C₆)-alkyle, (C₃-C₁₂)-cycloalkyle, (C₃-C₁₂)-cycloalkyle-(C₁-C₆)-alkyle et oxo, ledit alkylène pouvant être accolé au niveau de la liaison carbone-carbone à un carbocycle ou un hétérocycle renfermant 1 ou 2 atomes d'azotes, de 5 à 7 chaînons, saturé ou insaturé, non substitué ou substitué par R₃ ;
R₃ représente un groupement alkyle ou alkyloxy renfermant de 1 à 6 atomes de carbone ;
R₄ représente un atome d'hydrogène ou un groupement alkyle renfermant de 1 à 6 atomes de carbone ;
R₅ représente un atome d'hydrogène, un groupement CO₂R₆, SO₂R₆, SO₂NHR₆ ou SO₂NHCO₂R₆ dans lesquels R₆ est un radical (C₁-C₈)-alkyle ou naphtyle, non substitué ou substitué par R₃, un radical cycloalkyle renfermant de 3 à 12 atomes de carbone ou (C₃-C₁₂)-cycloalkyle-(C₁-C₆)-alkyle ou le radical de formule (II) 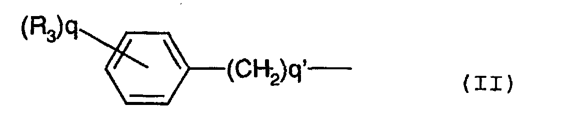 dans lequel les radicaux R₃ peuvent être identiques ou différents, et peuvent être localisés à n'importe quelle position du radical phényle, q et q' sont égaux à 0 ou 1 ;
m est un entier égal à 1, 2 ou 3 ;
n est un entier égal à 1, 2 ou 3 ;
lesdits radicaux alkyles pouvant être mono-ou poly-insaturés, lesdits radicaux cycloalkyles pouvant être mono, bi ou tricycliques non substitués ou substitués par un ou plusieurs (C₁-C₄)-alkyles ou pour les bi- et tricycloalkyles par un ou plusieurs oxos, lesdits radicaux aryles ou hétéroaryles pouvant être non substitués ou substitués par un ou plusieurs radicaux identiques ou différents choisis parmi (C₁-C₈)-alkyle, (C₁-C₈)-alkoxy, halogène, nitro, amino, trifluorométhyle, hydroxyle, méthylènedioxy, cyano, hydroxycarbonyle, aminocarbonyle, (C₁-C₄)-alkoxycarbonyle, phényle, phénoxy, benzyle et benzyloxy, lesdits composés de formule (I) étant sous toutes leurs formes stéréo isomères possibles, seuls ou en mélange selon un rapport quelconque, ainsi que leurs sels physiologiquement acceptables et leurs prodrugs.

4. un composé de formule (I') telle que définie à la revendication 2 ou 3, dans laquelle, R₁ et R₂ représentent un atome d'hydrogène ou forment ensemble un radical bivalent alkylène saturé ou insaturé renfermant de 2 à 3 atomes de carbone, ledit radical alkylène étant non substitué ou substitué par un ou deux radicaux identiques ou différents choisi parmi halogène, (C₁-C₆)-alkyle, (C₁-C₆)-alkoxy, (C₆-C₁₄)-aryle, (C₆-C₁₄)-aryle- (C₁-C₆)alkyle, (C₅-C₁₄)-hétéroaryle, (C₅-C₁₄)-hétéroaryle-(C₁-C₆)-alkyle, (C₃-C₁₂)-cycloalkyle, (C₃-C₁₂)-cycloalkyle-(C₁-C₆)-alkyle et oxo, ledit alkylène pouvant être accolé au niveau de la liaison carbone-carbone à un carbocycle ou un hétérocycle renfermant 1 ou 2 atomes d'azotes, à 5 à 7 chaînons, saturé ou insaturé, non substitué ou substitué par R₃ ;
R₃ représente un groupement alkyle ou alkyloxy renfermant de 1 à 6 atomes de carbone ;
R₄ représente un atome d'hydrogène ou un groupement alkyle renfermant de 1 à 6 atomes de carbone ;
R₅ représente un atome d'hydrogène, un groupement CO₂R₆, SO₂R₆, SO₂NHR₆ ou SO₂NHCO₂R₆ dans lesquels R₆ est un radical (C₁-C₈)-alkyle ou naphtyle, non substitué ou substitué par R₃, un radical cycloalkyle renfermant de 3 à 12 atomes de carbone ou (C₃-C₁₂)-cycloalkyle-(C₁-C₆)-alkyle ou le radical de formule (II) 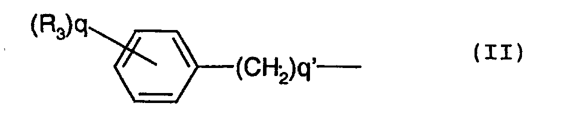 dans lequel les radicaux R₃ peuvent être identiques ou différents, et peuvent être localisés à n'importe quelle position du radical phényle, q et q' sont égaux à 0 ou 1 ;
m est un entier égal à 2 ;
n est un entier égal à 1 ;
lesdits radicaux alkyles pouvant être mono-ou poly-insaturés, lesdits radicaux cycloalkyles pouvant être mono, bi ou tricycliques non substitués ou substitués par un ou plusieurs (C₁-C₄)-alkyles ou pour les bi- et tricycloalkyles par un ou plusieurs oxos, lesdits radicaux aryles ou hétéroaryles pouvant être non substitués ou substitués par un ou plusieurs radicaux identiques ou différents choisis parmi (C₁-C₈)-alkyle, (C₁-C₈)-alkoxy, halogène, nitro, amino, trifluorométhyle, hydroxyle, méthylènedioxy, cyano, hydroxycarbonyle, aminocarbonyle, (C₁-C₄)-alkoxycarbonyle, phényle, phénoxy, benzyle et benzyloxy, lesdits composés de formule (I) étant sous toutes leurs formes stéréo isomères possibles, seuls ou en mélange selon un rapport quelconque, ainsi que leurs sels physiologiquement acceptables et leurs prodrugs.

5. Un composé de formules (I) ou (I') tel que défini à l'une quelconque des revendications 1 à 4 dans laquelle R₅ est un radical CO₂R₆, R₆ étant -CH₂Ph, -C(CH₃)₃ et CH₂-Adamantyle, lesdits composés de formule (I) étant sous toutes leurs formes stéréo isomères possibles, seuls ou en mélange selon un rapport quelconque, ainsi que leurs sels physiologiquement acceptables et leurs prodrugs.

6. Un composé de formule (I) ou (I') tel que défini à l'une quelconque des revendications 1 à 4 dans laquelle R₅ est un radical SO₂R₆, R₆ étant alkyle renfermant de 1 à 6 atomes de carbone, naphtyle et phényle substitué par un ou plusieurs radicaux alkyle renfermant de 1 à 6 atomes de carbone ou groupement CF₃, lesdits composés de formule (I) étant sous toutes leurs formes stéréo isomères possibles, seuls ou en mélange selon un rapport quelconque, ainsi que leurs sels physiologiquement acceptables et leurs prodrugs.

7. Un composé de formules (I) ou (I') tel que défini à l'une quelconque des revendications 1 à 4 dans laquelle R₅ est un radical SO₂NHR₆ ou SO₂NHCO₂R₆, R₆ étant -CH₂Ph, -C(CH₃)₃ et CH₂-Adamantyle, lesdits composés de formule (I) étant sous toutes leurs formes stéréo isomères possibles, seuls ou en mélange selon un rapport quelconque, ainsi que leurs sels physiologiquement acceptables et leurs prodrugs.

8. Un composé de formule (I) ou (I') tel que défini à 'une quelconque des revendications 1 à 7 dont les noms suivent
- Acide 5-[3-oxo-3-[(1,4,5,6-tétrahydro-2-pyrimidinyl)amino)-propyl]-.alpha.-[[(phénylméthoxy)carbonyl]amino]-2-benzofuranepropanoique,
- 5-[3-oxo-3-[(1,4,5,6-tétrahydro-2-pyrimidinyl)amino]-propyl-.alpha.-[[(phénylméthoxy)carbonyl]amino]-2-benzofuranpropanoate de 1-méthyléthyle,
- 5-[3-oxo-3-[(1,4,5,6-tétrahydro-2-pyrimidinyl)amino]-propyl]-.alpha.-[[(phénylméthoxy)carbonyl]amino]-2-benzofuranpropanoate d'éthyle,
- 5-[3-oxo-3-[(1,4,5,6-tétrahydro-2-pyrimidinyl)amino]-propyl]-.alpha.-[[[(tricyclo-[3.3.1.1^{3,7}]dec-1-yl)méthoxy]-carbonyl]amino]-2-benzofuranpropanoique,
- Acide 5-[3-oxo-3-[(1,4,5,6-tétrahydro-2-pyrimidinyl)-amino]propyl]-.alpha.-[[[[[(tricyclo[2.3.1.1^{3,7}]dec-1-yl)-méthoxy]carbonyl]amino]sulfonyl]amino]-2-benzofuranpropanoique,
- Acide 5-[3-oxo-3-[(1,4,5,6-tétrahydro-2-pyrimidinyl)amino]-propyl]-.alpha.-[[[[(phényl)méthoxy]carbonyl]-amino]-sulfonyl] amino]-2-benzofuranpropanoique,
- .alpha.-[[[(phénylméthyl)-amino]sulfonyl]amino]-5-[3-oxo-3-[(1,4,5,6-tétrahydro-2-pyrimidinyl)amino]propyl]-2-benzofuranpropanoique,
- Acide .alpha.-[[[4-(1,1-diméthyléthyl)phényl]sulfonyl]-amino]-5-(3-oxo-3-[(1,4,5,6-tétrahydro-2-pyrimidinyl)-amino]-propyl]-2-benzofuranpropanoique,
- Acide .alpha.-[[[4-(1-méthyléthyl)phényl]sulfonyl]amino]-5-(3-oxo-3-[(1,4,5,6-tétrahydro-2-pyrimidinyl)amino]propyl]-2-benzofuranpropanoique,
- Acide 5-[3-oxo-3-[(1,4,5,6-tétrahydro-2-pyrimidinyl)-amino]propyl]-.alpha.-[propylsulfonyl)amino]-2-benzofuranpropanoique,
- Acide.alpha.-[méthylsulfonyl)amino]-5-[3-oxo-3-[(1,4,5,6-tétrahydro-2-pyrimidinyl)amino]propyl]--2-benzofuranpropanoique,
- Acide.alpha.-[(1-naphtalenylsulfonyl)amino]-5-[3-oxo-3-[(1,4,5,6-tétrahydro-2-pyrimidinyl)amino]propyl]-2-benzofuranpropanoique,
- alpha.-[(1-naphtalenylsulfonyl)amino]-5-[3-oxo-3-[(1,4,5,6-tétrahydro-2-pyrimidinyl)amino]propyl]--2-benzofuranpropanoate de 1-méthyléthyle, ainsi que leurs sels physiologiquement acceptables et leurs prodrugs.

9. Un procédé de préparation des composés de formule (I) tels que définis à l'une quelconque des revendications 1 à 8, qui comprend le couplage de deux ou plusieurs fragments qui peuvent être dérivés par rétrosynthèse de composés de formule (I).

10. Un procédé selon la revendication 9 dans lequel on met en oeuvre les étapes suivantes :
a) formation d'un acide carboxylique ou un dérivé d'acide carboxylique de formule (III) 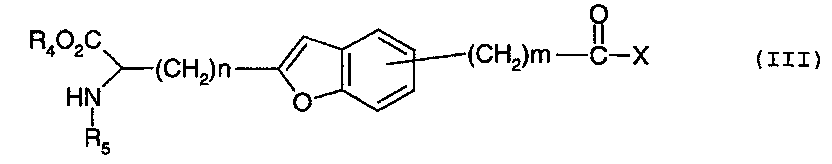 dans laquelle R₄, R₅, n et m sont tels que définis à la revendication 1 pour la formule (I) et dans laquelle X est un groupe partant substituable par un nucléophile, par action de l'amino ester de formule (V) 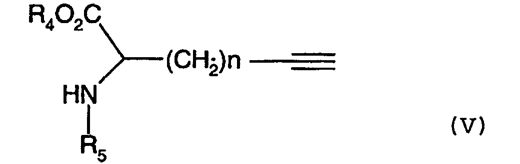 dans laquelle R₄, R₅, et n sont tels que définis à la revendication 1 pour la formule (I),
avec un acide carboxylique ou un dérivé d'acide carboxylique de formule (VI), en présence d'un catalyseur et en milieu basique 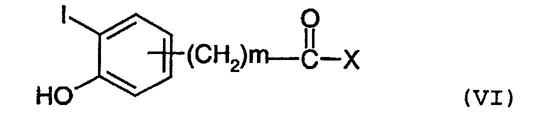 dans laquelle m et X sont tels que définis plus haut, les groupes fonctionnels éventuellement présents sous forme de précurseurs ou sous forme protégée, étant par la suite convertis en groupes présents dans les composés de formule (I) et
b)couplage de l'acide carboxylique ou un dérivé d'acide carboxylique de formule (III) telle que définie plus haut avec une guanidine ou un dérivé de la guanidine de formule (IV) 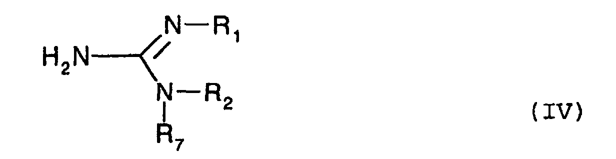
dans laquelle R₁, R₂ et R₇ sont tels que définis à la revendication 1 dans la formule (I), les groupes fonctionnels éventuellement présent sous forme de précurseurs ou sous forme protégée étant par la suite convertis en groupes présents dans les composés de formule (I).

11. A titre de médicament, un composé de formule (I) et/ou ses sels physiologiquement acceptables et/ou ses prodrugs tel que défini à l'une quelconque des revendications 1 à 8.

12. Une composition pharmaceutique comprenant au moins un composé de formule (I) tel que défini à l'une quelconque des revendications 1 à 8 et/ou ses sels physiologiquement acceptables et/ou ses prodrugs ainsi qu'un ou plusieurs supports usuels pharmaceutiquement inertes et le cas échéant un ou plusieurs additifs.

13. A titre de médicament ayant uns activité antagoniste du récepteur de la vitronectine, un composé de formule (I) et/ou ses sels physiologiquement acceptables et/ou ses prodrugs tel que défini à l'une quelconque des revendications 1 à 8.

14. A titre de médicament ayant une activité inhibitrice de la résorption osseuse ou pour le traitement ou la prévention de l'ostéoporose, un composé de formule (I) et/ou ses sels physiologiquement acceptables et/ou ses prodrugs tel que défini à l'une quelconque des revendications 1 à 8.

15. A titre de médicament ayant une activité inhibitrice de la croissance tumorale ou des métastases cancéreuses, un composé de formule (I) et/ou ses sels physiologiquement acceptables et/ou ses prodrugs tel que défini à l'une quelconque des revendications 1 à 8.

16. A titre de médicament ayant une activité anti-inflammatoire ou pour le traitement ou la prévention des désordres cardiovasculaires, la resténose, l'artériosclérose, les nephropathies ou rétinopathies, un composé de formule (I) et/ou ses sels physiologiquement acceptables et/ou ses prodrugs tel que défini à l'une quelconque des revendications 1 à 8.

17. Utilisation des composés de formule (I) et/ou leurs sels physiologiquement acceptables et/ou leurs prodrugs tels que définis à l'une quelconque des revendications 1 à 8 pour la préparation de médicaments destinés à la prévention ou au traitement de l'ostéoporose.

18. Utilisation des composés de formule (I) et/ou leurs sels physiologiquement acceptables et/ou leurs prodrugs tels que définis à l'une quelconque des revendications 1 à 8 pour la préparation de médicaments destinés à inhiber la croissance tumorale ou les métastases cancéreuses.

19. Utilisation des composés de formule (I) et/ou leurs sels physiologiquement acceptables et/ou leurs prodrugs tels que définis à Tune quelconque des revendications 1 à 8 pour la préparation de médicaments destinés à la prévention ou au traitement des désordres cardiovasculaires,de la resténose, de l'artériosclérose, des néphropathies ou rétinopathie.

## Claims

1. A compound of formula (I) 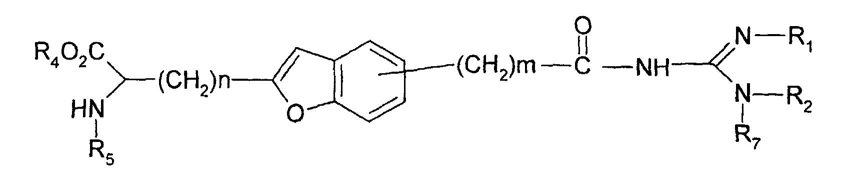 in which
either R₁ and R₂, independently from one another represent a hydrogen atom or an alkyl group containing 1 to 6 carbon atoms non-substituted or substituted by R₃,
or R₁ and R₂ together form a divalent alkylene radical containing 2 to 9 carbon atoms, saturated or unsaturated, non-substituted or substituted by one or more radicals chosen from halogen, (C₁-C₆)-alkyl, (C₁-C₆)-alkoxy, (C₆-C₁₄)-aryl, (C₆-C₁₄)-aryl-(C₁-C₆)-alkyl, (C₅-C₁₄)-heteroaryl, (C₅-C₁₄)-heteroaryl-(C₁-C₆)-alkyl, (C₃-C₁₂)-cycloalkyl (C₃-C₁₂)-cycloalkyl-(C₁-C₆)-alkyl and oxo, said divalent alkylene radical being able to be attached at the level of the carbon-carbon bond to a carbocycle or a heterocycle with 5 to 7 members, containing 1 or 2 nitrogen atoms, saturated or unsaturated, non-substituted or substituted by 1 or 2 R₃ radicals;
R₃ represents a (C₁-C₈)-alkyl, (C₁-C₈)-alkoxy, (C₆-C₁₄)-aryl, (C₆-C₁₄)-aryl- (C₁-C₄)-alkyl, (C₅-C₁₄) -heteroaryl, (C₅-C₁₄)-heteroaryl-(C₁-C₄)-alkyl, halogen, trifluoromethyl, hydroxyl, nitro, amino, NH-((C₁-C₄)-alkyl),N((C₁-C₄)alkyl)₂, NHCO-(C₁-C₄)-alkyl or CO-(C₁-C₄)alkyl group;
R₄ represents
either a hydrogen atom, or a (C₁-C₆)-alkyl-CO-O-(C₁-C₄)-alkyl or (C₁-C₆)-alkyl group, non-substituted or substituted by a radical chosen from hydroxyl, (C₁-C₄)-alkoxy, (C₁-C₄)-alkyl-SO₂, NR₉R₉' and N⁺R₉R₉'R₉"Q⁻, in which R₉, R₉' and R₉" independently from one another, represent a hydrogen, a (C₁-C₆)-alkyl, (C₆-C₁₄)-aryl, (C₆-C₁₄)-aryl-(C₁-C₆)-alkyl, (C₅-C₁₄)-heteroaryl, (C₅-C₁₄)-heteroaryl-(C₁-C₄)-alkyl group and Q⁻ is a physiologically acceptable anion,
or one of the following radicals: 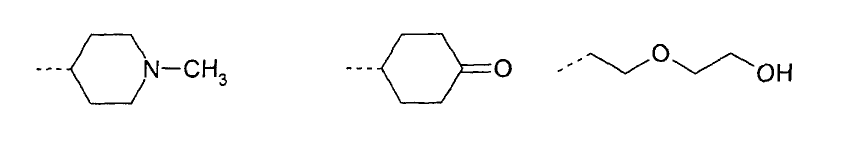 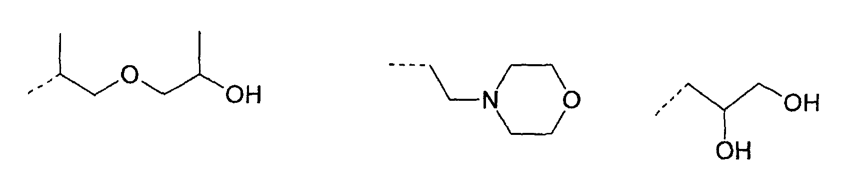 the dotted lines representing the position of the bond;
R₅ represents a hydrogen atom or a group chosen from COR₆, CO₂R₆, SO₂R₆, SO₂NHR₆, SO₂NHCOR₆, SO₂NHCO₂R₆, CONH₂ and CONHR₆ in which R₆ represents (C₁-C₈) -alkyl, (C₆-C₁₄) -aryl, (C₆-C₁₄) -aryl-(C₁-C₆)-alkyl, (C₅-C₁₄)-heteroaryl or (C₅-C₁₄)-heteroaryl-(C₁-C₆)-alkyl, (C₃-C₂₀) (mono-, bi- or tri-)-cycloalkyl, (C₃-C₂₀) (mono-, bi- or tri-)-cycloalkyl-(C₁-C₆)alkyl, the aryl or heteroaryl radical being non-substituted or substituted by 1, 2 or 3 R₃ radicals;
R₇ represents a hydrogen atom, (C₁-C₆)-alkyl-O-CO-, hydroxyl, (C₁-C₆)-alkyl-O-CO-O or nitro;
m is equal to 0, 1, 2 or 3;
n is an integer equal to 1, 2 or 3;
said compounds being able to be mono- or polyunsaturated, said cycloalkyl radicals being able to be mono-, bi- or tricyclic not substituted or substituted by one or more (C₁-C₄)-alkyls or for the bi- and tricycloalkyls by one or more oxos, said aryl or heteroaryl radicals being able to be not substituted or substituted by one or more identical or different radicals chosen from (C₁-C₈)-alkyl, (C₁-C₈)-alkoxy, halogen, nitro, amino, trifluoromethyl, hydroxyl, methylenedioxy, cyano, hydroxycarbonyl, aminocarbonyl, (C₁-C₄)-alkoxycarbonyl, phenyl, phenoxy, benzyl and benzyloxy, said compounds of formula (I) being in all their possible stereoisomer forms, alone or in a mixture in any ratio, the acylguanidine group adjacent to the phenyl being in para or meta position of the oxygen, as well as their physiologically acceptable salts and their prodrugs.

2. A compound of formula (I) as defined in claim 1 corresponding to formula (I') 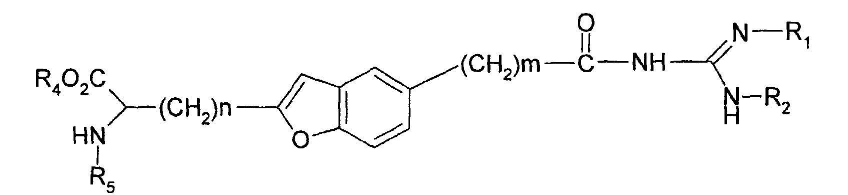 in which, R₁ and R₂ represent a hydrogen atom or together form a saturated or unsaturated divalent alkylene radical containing 2 to 5 carbon atoms, said alkylene radical being non-substituted or substituted by one or two identical or different radicals chosen from halogen, (C₁-C₆)-alkyl, (C₁-C₆)-alkoxy, (C₆-C₁₄)-aryl, (C₆-C₁₄)-aryl-(C₁-C₆)-alkyl, (C₅-C₁₄)-heteroaryl, (C₅-C₁₄)-heteroaryl-(C₁-C₆)-alkyl, (C₃-C₁₂)-cycloalkyl and (C₃-C₁₂)-cycloalkyl-(C₁-C₆)-alkyl and oxo, said alkylene being able to be attached at the level of the carbon-carbon bond to a carbocycle or a heterocycle containing 1 or 2 nitrogen atoms, with 5 to 7 members, saturated or unsaturated, non-substituted or substituted by R₃;
R₃ represents an alkyl or alkyloxy group containing 1 to 6 carbon atoms; R₄ represents a hydrogen atom or an alkyl group containing 1 to 6 carbon atoms non-substituted or substituted by a group chosen from (C₁-C₄)-alkoxy, (C₁-C₄)-alkyl-SO₂ and NR₉R₉' in which R₉ and R₉' independently from one another represent a hydrogen atom or (C₁-C₄)-alkyl,
R₅ represents a hydrogen atom, a CO₂R₆, SO₂R₆, SO₂NHR₆ or SO₂NHCO₂R₆ group in which R₆ is a (C₁-C₈)-alkyl or naphthyl radical, non-substituted or substituted by R₃, a cycloalkyl radical containing 3 to 12 carbon atoms or (C₃-C₁₂)-cycloalkyl-(C₁-C₆)-alkyl or the radical of formula (II) 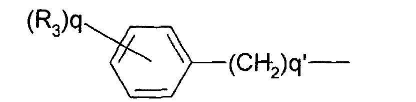 in which the R₃ radicals can be identical or different, and can be situated at any position of the phenyl radical, q and
q' are equal to 0 or 1;
m is an integer equal to 1, 2 or 3;
n is an integer equal to 1, 2 or 3,
said alkyl radicals being able to be mono- or polyunsaturated, said cycloalkyl radicals being able to be mono-, bi- or tricyclic not substituted or substituted by one or more (C₁-C₄)-alkyls or for the bi- and tricycloalkyls by one or more oxos, said aryl or heteroaryl radicals being able to be not substituted or substituted by one or more identical or different radicals chosen from (C₁-C₈)-alkyl, (C₁-C₈)-alkoxy, halogen, nitro, amino, trifluoromethyl, hydroxyl, methylenedioxy, cyano, hydroxycarbonyl, aminocarbonyl, (C₁-C₄)-alkoxycarbonyl, phenyl, phenoxy, benzyl and benzyloxy, said compounds of formula (I') being in all their possible stereoisomer forms, alone or in a mixture in any ratio, as well as their physiologically acceptable salts and their prodrugs.

3. A compound of formula (I') as defined in claim 2 in which R₁ and R₂ represent a hydrogen atom, or together form a saturated or unsaturated divalent alkylene radical containing 2 to 4 carbon atoms, said alkylene radical being non-substituted or substituted by one or two identical or different radicals chosen from halogen, (C₁-C₆)-alkyl, (C₁-C₆)-alkoxy, (C₆-C₁₄)-aryl, (C₆-C₁₄)-aryl-(C₁-C₆)-alkyl, (C₅-C₁₄)-heteroaryl, (C₅-C₁₄)-heteroaryl- (C₁-C₆)-alkyl, (C₃-C₁₂)-cycloalkyl and (C₃-C₁₂)-cycloalkyl-(C₁-C₆)-alkyl and oxo, said alkylene being able to be attached at the level of the carbon-carbon bond to a carbocycle or a heterocycle containing 1 or 2 nitrogen atoms, with 5 to 7 members, saturated or unsaturated, non-substituted or substituted by R₃;
R₃ represents an alkyl or alkyloxy group containing 1 to 6 carbon atoms;
R₄ represents a hydrogen atom or an alkyl group containing 1 to 6 carbon atoms;
R₅ represents a hydrogen atom, a CO₂R₆, SO₂R₆, SO₂NHR₆ or SO₂NHCO₂R₆ group in which R₆ is a (C₁-C₈)-alkyl or naphthyl radical, non-substituted or substituted by R₃, a cycloalkyl radical containing 3 to 12 carbon atoms or (C₃-C₁₂)-cycloalkyl-(C₁-C₆)alkyl or the radical of formula (II) 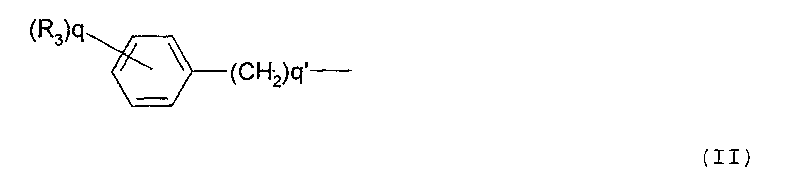 in which the R₃ radicals can be identical or different, and can be situated at any position of the phenyl radical, q and
q' are equal to 0 or 1;
m is an integer equal to 1, 2 or 3;
n is an integer equal to 1, 2 or 3;
said alkyl radicals being able to be mono- or polyunsaturated, said cycloalkyl radicals being able to be mono-, bi- or tricyclic not substituted or substituted by one or more (C₁-C₄)-alkyls or for the bi- and tricycloalkyls by one or more oxos, said aryl or heteroaryl radicals being able to be not substituted or substituted by one or more identical or different radicals chosen from (C₁-C₈)-alkyl, (C₁-C₈)-alkoxy, halogen, nitro, amino, trifluoromethyl, hydroxyl, methylenedioxy, cyano, hydroxycarbonyl, aminocarbonyl, (C₁-C₄)-alkoxycarbonyl, phenyl, phenoxy, benzyl and benzyloxy,said compounds of formula (I) being in all their possible stereoisomer forms, alone or in a mixture in any ratio, as well as their physiologically acceptable salts and their prodrugs.

4. A compound of formula (I') as defined in claim 2 or 3, in which R₁ and R₂ represent a hydrogen atom, or together form a saturated or unsaturated divalent alkylene radical containing 2 to 3 carbon atoms, said alkylene radical being non-substituted or substituted by one or two identical or different radicals chosen from halogen, (C₁-C₆)-alkyl, (C₁-C₆)-alkoxy, (C₆-C₁₄)-aryl, (C₆-C₁₄)-aryl-(C₁-C₆)alkyl, (C₅-C₁₄)-heteroaryl, (C₅-C₁₄)-heteroaryl- (C₁-C₆)-alkyl, (C₃-C₁₂)-cycloalkyl, (C₃-C₁₂)-cycloalkyl-(C₁-C₆)-alkyl and oxo, said alkylene being able to be attached at the level of the carbon-carbon bond to a carbocycle or a heterocycle containing 1 or 2 nitrogen atoms, with 5 to 7 members, saturated or unsaturated, non-substituted or substituted by R₃;
R₃ represents an alkyl or alkyloxy group containing 1 to 6 carbon atoms;
R₄ represents a hydrogen atom or an alkyl group containing 1 to 6 carbon atoms;
R₅ represents a hydrogen atom, a CO₂R₆, SO₂R₆, SO₂NHR₆ or SO₂NHCO₂R₆ group in which R₆ is a (C₁-C₈)-alkyl or naphthyl radical, non-substituted or substituted by R₃, a cycloalkyl radical containing 3 to 12 carbon atoms or (C₃-C₁₂)-cycloalkyl-(C₁-C₆)-alkyl or the radical of formula (II) 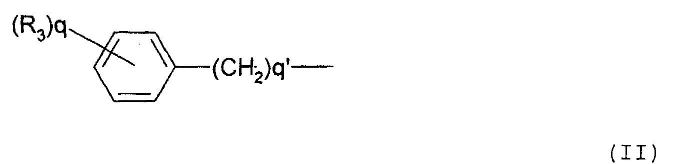 in which the R₃ radicals can be identical or different, and can be situated at any position of the phenyl radical, q and
q' are equal to 0 or 1;
m is an integer equal to 2;
n is an integer equal to 1;
said alkyl radicals being able to be mono- or polyunsaturated, said cycloalkyl radicals being able to be mono-, bi- or tricyclic not substituted or substituted by one or more (C₁-C₄)-alkyls or for the bi- and tricycloalkyls by one or more oxos, said aryl or heteroaryl radicals being able to be not substituted or substituted by one or more identical or different radicals chosen from (C₁-C₈)-alkyl, (C₁-C₈)-alkoxy, halogen, nitro, amino, trifluoromethyl, hydroxyl, methylenedioxy, cyano, hydroxycarbonyl, aminocarbonyl, (C₁-C₄)-alkoxycarbonyl, phenyl, phenoxy, benzyl and benzyloxy said compounds of formula (I) being in all their possible stereoisomer forms, alone or in a mixture in any ratio, as well as their physiologically acceptable salts and their prodrugs.

5. A compound of formulae (I) or (I') as defined in any one of claims 1 to 4 in which R₅ is a CO₂R₆ radical, R₆ being -CH₂Ph, -C(CH₃)₃ and CH₂-Adamantyl, said compounds of formula (I) being in all their possible stereoisomer forms, alone or in a mixture in any ratio, as well as their physiologically acceptable salts and their prodrugs.

6. A compound of formula (I) or (I') as defined in any one of claims 1 to 4 in which R₅ is an SO₂R₆ radical, R₆ being an alkyl containing 1 to 6 carbon atoms, naphthyl and phenyl substituted by one or more alkyl radicals containing 1 to 6 carbon atoms, or a CF₃ group, said compounds of formula (I) being in all their possible stereoisomer forms, alone or in a mixture in any ratio, as well as their physiologically acceptable salts and their prodrugs.

7. A compound of formula (I) or (I') as defined in any one of claims 1 to 4 in which R₅ is an SO₂NHR₆ or SO₂NHCO₂R₆ radical, R₆ being -CH₂Ph, -C(CH₃)₃ and CH₂-Adamantyl, said compounds of formula (I) being in all their possible stereoisomer forms, alone or in a mixture in any ratio, as well as their physiologically acceptable salts and their prodrugs.

8. A compound of formula (I) or (I') as defined in any one of claims 1 to 7 the names of which follow:
- 5-[3-oxo-3-[(1,4,5,6-tetrahydro-2-pyrimidinyl)amino)-propyl]-.alpha.-[[(phenylmethoxy)carbonyl]amino]-2-benzofuranepropanoic acid,
- 1-methylethyl 5-[3-oxo-3-[(1,4,5,6-tetrahydro-2-pyrimidinyl)amino]-propyl-.alpha.-[[(phenylmethoxy) carbonyl]amino]-2-benzo-furanpropanoate,
- ethyl 5-[3-oxo-3-[(1,4,5,6-tetrahydro-2-pyrimidinyl)amino]-propyl]-.alpha.-[[(phenylmethoxy)carbonyl]amino]-2-benzofuranpropanoate,
- 5-[3-oxo-3-[(1,4,5,6-tetrahydro-2-pyrimidinyl)amino]-propyl]-.alpha.-[[[(tricyclo-[3.3.1.1^{3,7}]dec-1-yl)methoxy]-carbonyl]amino]-2-benzofuranpropanoic,
- 5-[3-oxo-3-[(1,4,5,6-tetrahydro-2-pyrimidinyl)-amino]propyl]-.alpha.-[[[[[(tricyclo[2.3.1.1^{3,7}]dec-1-yl)-methoxy]carbonyl]amino]sulphonyl]amino]-2-benzofuranpropanoic acid,
- 5-[3-oxo-3-[(1,4,5,6-tetrahydro-2-pyrimidinyl)amino]-propyl]-.alpha.-[[[[(phenyl)methoxy]carbonyl]-amino]-sulphonyl] amino]-2-benzofuranpropanoic acid,
- .alpha.-[[[(phenylmethyl)-amino]sulphonyl]amino]-5-[3-oxo-3-[(1,4,5,6-tetrahydro-2-pyrimidinyl)amino]propyl]-2-benzofuranpropanoic,
- .alpha.-[[[4-(1,1-dimethylethyl)phenyl]sulphonyl]-amino]-5-(3-oxo-3-[(1,4,5,6-tetrahydro-2-pyrimidinyl)-amino]-propyl]-2-benzofuranpropanoic acid,
- .alpha.-[[[4-(1-methylethyl)phenyl]sulphonyl]amino]-5-(3-oxo-3-[(1,4,5,6-tetrahydro-2-pyrimidinyl)amino]propyl] -2-benzofuranpropanoic acid,
- 5-[3-oxo-3-[(1,4,5,6-tetrahydro-2-pyrimidinyl)-amino]propyl]-.alpha.-[propylsulphonyl)amino]-2-benzofuranpropanoic acid,
- .alpha.-[methylsulphonyl)amino]-5-[3-oxo-3-[(1,4,5,6-tetrahydro-2-pyrimidinyl)amino]propyl]--2-benzofuranpropanoic acid,
- .alpha.-[(1-naphthalenylsulphonyl)amino]-5-[3-oxo-3-[(1,4,5,6-tetrahydro-2-pyrimidinyl)amino]propyl]-2-benzofuranpropanoic acid,
- 1-methylethyl alpha.-[(1-naphthalenylsulphonyl)amino]-5-[3-oxo-3-[(1,4,5,6-tetrahydro-2-pyrimidinyl)amino]propyl]--2-benzofuranpropanoate,
as well as their physiologically acceptable salts and their prodrugs.

9. A process for the preparation of the compounds of formula (I) as defined in any one of claims 1 to 8, which comprises the coupling of two or more fragments which can be derived by retrosynthesis from the compounds of formula (I).

10. A process according to claim 9 in which the following stages are implemented:
a) formation of a carboxylic acid or a carboxylic acid derivative of formula (III) 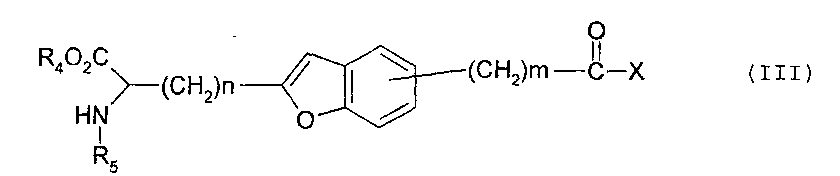 in which R₄, R₅, n and m are as defined in claim 1 for formula (I) and in which X is a parting group which can be substituted by a nucleophile,
by the action of the amino ester of formula (V) 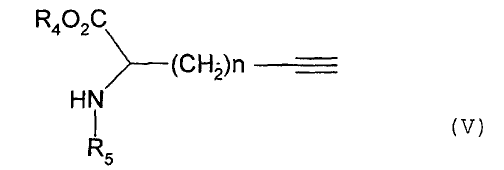 in which R₄, R₅ and n are as defined in claim 1, for formula (I),
with a carboxylic acid or a carboxylic acid derivative of formula (VI), in the presence of a catalyst and in a basic medium 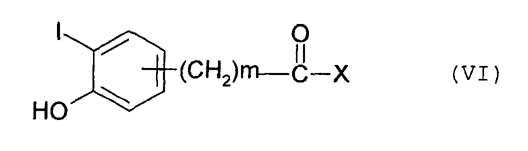 in which m and X are as defined above, the functional groups optionally present in the form of precursors or in protected form, being subsequently converted to groups present in the compounds of formula (I) and
b) coupling of the carboxylic acid or a carboxylic acid derivative of formula (III) as defined above with a guanidine or a guanidine derivative of formula (IV) 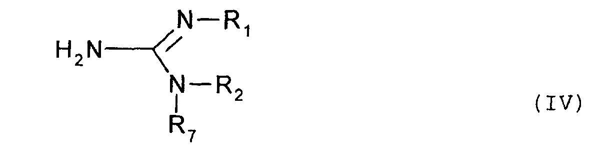 in which R₁, R₂ and R₇, are as defined in claim 1 in formula (I), the functional groups optionally present in the form of precursors or in protected form being subsequently converted to groups present in the compounds of formula (I).

11. As a medicament, a compound of formula (I) and/or its physiologically acceptable salts and/or its prodrugs as defined in any one of claims 1 to 8.

12. A pharmaceutical composition comprising at least one compound of formula (I) as defined in any one of claims 1 to 8 and/or its physiologically acceptable salts and/or its prodrugs as well as one or more usual pharmaceutically inert supports and if appropriate one or more additives.

13. As a medicament having an antagonist activity on the vitronectin receptor, a compound of formula (I) and/or its physiologically acceptable salts and/or its prodrugs as defined in any one of claims 1 to 8.

14. As a medicament having an inhibitory activity on bone resorption or for the treatment or prevention of osteoporosis, a compound of formula (I) and/or its physiologically acceptable salts and/or its prodrugs as defined in any one of claims 1 to 8.

15. As a medicament having an inhibitory activity on tumorous growth or cancerous metastases, a compound of formula (I) and/or its physiologically acceptable salts and/or its prodrugs as defined in any one of claims 1 to 8.

16. As a medicament having an anti-inflammatory activity or for the treatment or prevention of cardiovascular disorders, the recurrence of stenosis, arteriosclerosis, nephropathies or retinopathies, a compound of formula (I) and/or its physiologically acceptable salts and/or its prodrugs as defined in any one of claims 1 to 8.

17. Use of the compounds of formula (I) and/or their physiologically acceptable salts and/or their prodrugs as defined in any one of claims 1 to 8 for the preparation of medicaments intended for the prevention or treatment of osteoporosis.

18. Use of the compounds of formula (I) and/or their physiologically acceptable salts and/or their prodrugs as defined in any one of claims 1 to 8 for the preparation of medicaments intended to inhibit tumorous growth or cancerous metastases.

19. Use of the compounds of formula (I) and/or their physiologically acceptable salts and/or their prodrugs as defined in any one of claims 1 to 8 for the preparation of medicaments intended for the prevention or treatment of cardiovascular disorders, the recurrence of stenosis, arteriosclerosis, nephropathies or retinopathies.

## Patentansprüche

1. Verbindung der Formel (I) 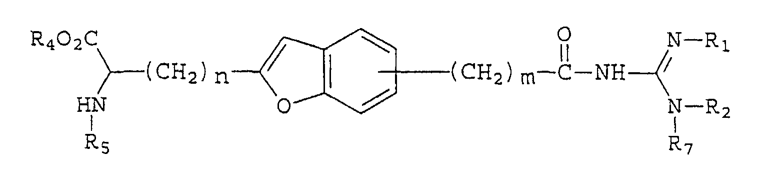 in der
entweder R₁ und R₂ unabhängig voneinander ein Wasserstoffatom oder eine Gruppe Alkyl mit 1 bis 6 Kohlenstoffatomen darstellen, nicht substituiert oder substituiert durch R₃,
oder R₁ und R₂ bilden zusammen einen gesättigten oder ungesättigten, bivalenten Rest Alkylen mit 2 bis 9 Kohlenstoffatomen, nicht substituiert oder substituiert durch einen oder mehrere Reste, ausgewählt unter Halogen, (C₁-C₆)-Alkyl, (C₁-C₆)-Alkoxy, (C₆-C₁₄)-Aryl, (C₆-C₁₄) -Aryl- (C₁-C₆)-alkyl, (C₅-C₁₄)-Heteroaryl, (C₅-C₁₄)-Heteroaryl- (C₁-C₆)-alkyl, (C₃-C₁₂)-Cycloalkyl, (C₃-C₁₂)-Cycloalkyl-(C₁-C₆)-alkyl und Oxo, wobei der bivalente Rest Alkylen im Bereich der Kohlenstoff-Kohlenstoff-Bindung mit einem Carbocyclus oder einem Heterocyclus mit 5 bis 7 Ringgliedern und 1 oder 2 Stickstoffatomen, gesättigt oder ungesättigt und nicht substituiert oder substituiert durch 1 oder 2 Reste R₃, verbunden sein kann;
R₃ eine Gruppe (C₁-C₈)-Alkyl, (C₁-C₈)-Alkoxy, (C₆-C₁₄)-Aryl, (C₆-C₁₄)]-Aryl-(C₁-C₄)-alkyl, (C₅-C₁₄)-Heteroaryl, (C₅-C₁₄)-Heteroaryl-(C₁-C₄)-alkyl, Halogen, Trifluormethyl, Hydroxyl, Nitro, Amino,
NH- [(C₁-C₄)-Alkyl], N[(C₁-C₄)-Alkyl]₂, NHCO-(C₁-C₄)-Alkyl oder CO- (C₁-C₄)-Alkyl bedeutet;
entweder R₄ ein Wasserstoffatom oder eine Gruppe (C₁-C₆)-Alkyl-CO-O-(C₁-C₄)-alkyl oder (C₁-C₆)-Alkyl darstellt, nicht substituiert oder substituiert durch einen Rest, ausgewählt unter Hydroxyl, (C₁-C₄)-Alkoxy, (C₁-C₄)-Alkyl-SO₂, NR₉R₉' und N⁺R₉R₉'R₉"Q⁻, worin R₉, R₉' und R₉", unabhängig voneinander, Wasserstoff, eine Gruppe (C₁-C₆)-Alkyl, (C₆-C₁₄)-Aryl, (C₆-C₁₄)-Aryl-(C₁-C₄)-alkyl, (C₅-C₁₄)-Heteroaryl, (C₅-C₁₄)-Heteroaryl-(C₁-C₄)-alkyl bedeuten und Q⁻ ein physiologisch akzeptables Anion ist,
oder einen der folgenden Reste darstellt: 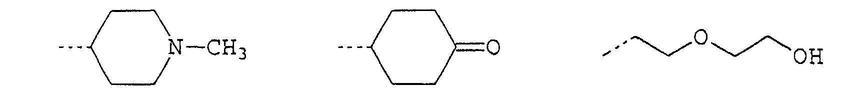 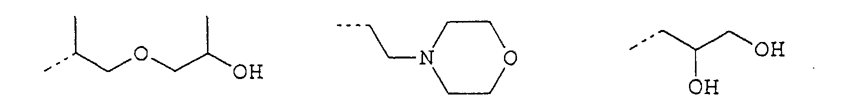 wobei die punktierten Linien die Position der Bindung darstellen; R₅ ein Wasserstoffatom oder eine Gruppe bedeutet, ausgewählt unter COR₆, CO₂R₆, SO₂R₆, SO₂NHR₆, SO₂NHCOR₆, SO₂NHCO₂R₆, CONH₂ und CONHR₆, worin R₆ (C₁-C₈)-Alkyl, (C₆-C₁₄)-Aryl, (C₆-C₁₄)-Aryl- (C₁-C₆)-alkyl, (C₅-C₁₄)-Heteroaryl oder (C₅-C₁₄)-Heteroaryl-(C₁-C₆)-alkyl, (C₃-C₂₀)-(Mono-, Bi- oder Tri-)-cycloalkyl, (C₃-C₂₀)-(Mono-, Bi- oder Tri-)-cycloalkyl-(C₁-C₆)-alkyl darstellt, wobei der Rest Aryl oder Heteroaryl nicht substituiert oder substituiert ist durch 1, 2 oder 3 Reste R₃;
R₇ ein Wasserstoffatom, (C₁-C₆)-Alkyl-O-CO-, Hydroxyl, (C₁-C₆)-Alkyl-O-CO-O- oder Nitro darstellt;
m gleich 0, 1, 2 oder 3 ist;
n eine ganze Zahl von 1, 2 oder 3 ist;
wobei die genannten Reste Alkyl mono- oder polyungesättigt, die genannten Reste Cycloalkyl mono-, bi- oder tricyclisch, nicht substituiert oder substituiert durch ein oder mehrere (C₁-C₄)-Alkyl oder bei den Bi- und Tricycloalkylen durch ein oder mehrere Oxo, sein können, die genannten Reste Aryl oder Heteroaryl nicht substituiert oder substituiert durch ein oder mehrere gleiche oder verschiedene Reste, ausgewählt unter (C₁-C₈)-Alkyl, (C₁-C₈)-Alkoxy, Halogen, Nitro, Amino, Trifluormethyl, Hydroxyl, Methylendioxy, Cyano, Hydroxycarbonyl, Aminocarbonyl, (C₁-C₄)-Alkoxycarbonyl, Phenyl, Phenoxy, Benzyl und Benzyloxy, sein können,
wobei die genannten Verbindungen der Formel (I) in allen ihren möglichen stereoisomeren Formen, allein oder in Mischung in irgendeinem Verhältnis, vorliegen können, und die an das Phenyl angrenzende Gruppe Acylguanidin sich in Position para oder meta vom Sauerstoff befindet,
sowie ihre physiologisch akzeptablen Salze und ihre Vormedikamente (Prodrugs).

2. Verbindung der Formel (I) wie in Anspruch 1 definiert, die der Formel (I') 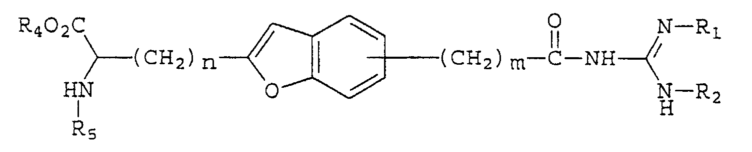 entspricht, in der
R₁ und R₂ ein Wasserstoffatom darstellen oder zusammen einen gesättigten oder ungesättigten, bivalenten Rest Alkylen mit 2 bis 5 Kohlenstoffatomen bilden, der nicht substituiert oder substituiert ist durch einen oder zwei, gleiche oder verschiedene Reste, ausgewählt unter Halogen, (C₁-C₆)-Alkyl, (C₁-C₆)-Alkoxy, (C₆-C₁₄)-Aryl, (C₆-C₁₄) -Aryl- (C₁-C₆)-alkyl, (C₅-C₁₄)-Heteroaryl, (C₅-C₁₄)-Heteroaryl-(C₁-C₆)-alkyl, (C₃-C₁₂)-Cycloalkyl und (C₃-C₁₂)-Cycloalkyl-(C₁-C₆)-alkyl und Oxo, wobei der Rest Alkylen im Bereich der Kohlenstoff-Kohlenstoff-Bindung mit einem Carbocyclus oder einem Heterocyclus mit 5 bis 7 Ringgliedern und 1 oder 2 Stickstoffatomen, gesättigt oder ungesättigt und nicht substituiert oder substituiert durch R₃, verbunden sein kann;
R₃ eine Gruppe Alkyl oder Alkoxy mit 1 bis 6 Kohlenstoffatomen bedeutet;
R₄ ein Wasserstoffatom oder eine Gruppe Alkyl mit 1 bis 6 Kohlenstoffatomen darstellt, nicht substituiert oder substituiert durch eine Gruppe, ausgewählt unter (C₁-C₄)-Alkoxy, (C₁-C₄)-Alkyl-SO₂ und NR₉R₉', worin R₉ und R₉', unabhängig voneinander, ein Wasserstoffatom oder (C₁-C₄)-Alkyl bedeuten;
R₅ ein Wasserstoffatom, eine Gruppe CO₂R₆, SO₂R₆, SO₂NHR₆ oder SO₂NHCO₂R₆ bedeutet, worin R₆ einen Rest (C₁-C₈)-Alkyl oder Naphthyl, nicht substituiert oder substituiert durch R₃, einen Rest Cycloalkyl mit 3 bis 12 Kohlenstoffatomen oder (C₃-C₁₂)-Cycloalkyl-(C₁-C₆)-alkyl oder den Rest der Formel (II) 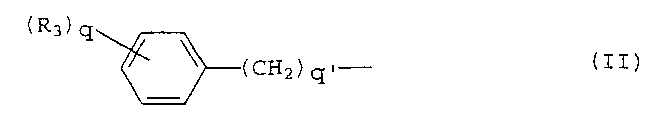 darstellt, worin die Reste R₃ gleich oder verschieden sein können und sich in irgendeiner Position des Restes Phenyl befinden können,
q und q' gleich 0 oder 1 sind;
m eine ganze Zahl von 1, 2 oder 3 ist;
n eine ganze Zahl von 1, 2 oder 3 ist;
wobei die genannten Reste Alkyl mono- oder polyungesättigt, die genannten Reste Cycloalkyl mono-, bi- oder tricyclisch, nicht substituiert oder substituiert durch ein oder mehrere (C₁-C₄)-Alkyl oder bei den Bi- und Tricycloalkylen durch ein oder mehrere Oxo, sein können, die genannten Reste Aryl oder Heteroaryl nicht substituiert oder substituiert durch ein oder mehrere gleiche oder verschiedene Reste, ausgewählt unter (C₁-C₈)-Alkyl, (C₁-C₈)-Alkoxy, Halogen, Nitro, Amino, Trifluormethyl, Hydroxyl, Methylendioxy, Cyano, Hydroxycarbonyl, Aminocarbonyl, (C₁-C₄)-Alkoxycarbonyl, Phenyl, Phenoxy, Benzyl und Benzyloxy, sein können,
wobei die genannten Verbindungen der Formel (I) in allen ihren möglichen stereoisomeren Formen, allein oder in Mischung in irgendeinem Verhältnis, vorliegen können, sowie ihre physiologisch akzeptablen Salze und ihre Prodrugs.

3. Verbindung der Formel (I') wie in Anspruch 2 definiert, in der R₁ und R₂ ein Wasserstoffatom darstellen oder zusammen einen gesättigten oder ungesättigten, bivalenten Rest Alkylen mit 2 bis 4 Kohlenstoffatomen bilden, der nicht substituiert oder substituiert ist durch einen oder zwei, gleiche oder verschiedene Reste, ausgewählt unter Halogen, (C₁-C₆)-Alkyl, (C₁-C₆)-Alkoxy, (C₆-C₁₄)-Aryl, (C₆-C₁₄) -Aryl-(C₁-C₆)-alkyl, (C₅-C₁₄)-Heteroaryl, (C₅-C₁₄)-Heteroaryl-(C₁-C₆)-alkyl, (C₃-C₁₂)-Cycloalkyl und (C₃-C₁₂)-Cycloalkyl-(C₁-C₆)-alkyl und Oxo, wobei der Rest Alkylen im Bereich der Kohlenstoff-Kohlenstoff-Bindung mit einem Carbocyclus oder einem Heterocyclus mit 5 bis 7 Ringgliedern und 1 oder 2 Stickstoffatomen, gesättigt oder ungesättigt und nicht substituiert oder substituiert durch R₃, verbunden sein kann;
R₃ eine Gruppe Alkyl oder Alkoxy mit 1 bis 6 Kohlenstoffatomen bedeutet;
R₄ ein Wasserstoffatom oder eine Gruppe Alkyl mit 1 bis 6 Kohlenstoffatomen darstellt,
R₅ ein Wasserstoffatom, eine Gruppe CO₂R₆, SO₂R₆, SO₂NHR₆ oder SO₂NHCO₂R₆ bedeutet, worin R₆ einen Rest (C₁-C₈)-Alkyl oder Naphthyl, nicht substituiert oder substituiert durch R₃, einen Rest Cycloalkyl mit 3 bis 12 Kohlenstoffatomen oder (C₃-C₁₂)-Cycloalkyl-(C₁-C₆)-alkyl oder den Rest der Formel (II) 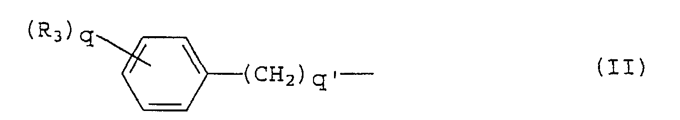 darstellt, worin die Reste R₃ gleich oder verschieden sein können und sich in irgendeiner Position des Restes Phenyl befinden können,
q und q' gleich 0 oder 1 sind;
m eine ganze Zahl von 1, 2 oder 3 ist;
n eine ganze Zahl von 1, 2 oder 3 ist;
wobei die genannten Reste Alkyl mono- oder polyungesättigt, die genannten Reste Cycloalkyl mono-, bi- oder tricyclisch, nicht substituiert oder substituiert durch ein oder mehrere (C₁-C₄)-Alkyl oder bei den Bi- und Tricycloalkylen durch ein oder mehrere Oxo, sein können, die genannten Reste Aryl oder Heteroaryl nicht substituiert oder substituiert durch ein oder mehrere gleiche oder verschiedene Reste, ausgewählt unter (C₁-C₈)-Alkyl, (C₁-C₈)-Alkoxy, Halogen, Nitro, Amino, Trifluormethyl, Hydroxyl, Methylendioxy, Cyano, Hydroxycarbonyl, Aminocarbonyl, (C₁-C₄)-Alkoxycarbonyl, Phenyl, Phenoxy, Benzyl und Benzyloxy, sein können,
wobei die genannten Verbindungen der Formel (I) in allen ihren möglichen stereoisomeren Formen, allein oder in Mischung in irgendeinem Verhältnis, vorliegen können, sowie ihre physiologisch akzeptablen Salze und ihre Prodrugs.

4. Verbindung der Formel (I') wie in Anspruch 2 oder 3 definiert, in der R₁ und R₂ ein Wasserstoffatom darstellen oder zusammen einen gesättigten oder ungesättigten, bivalenten Rest Alkylen mit 2 bis 3 Kohlenstoffatomen bilden, der nicht substituiert oder substituiert ist durch einen oder zwei, gleiche oder verschiedene Reste, ausgewählt unter Halogen, (C₁-C₆)-Alkyl, (C₁-C₆)-Alkoxy, (C₆-C₁₄)-Aryl, (C₆-C₁₄)-Aryl-(C₁-C₆)-alkyl, (C₅-C₁₄)-Heteroaryl, (C₅-C₁₄)-Heteroaryl-(C₁-C₆)-alkyl, (C₃-C₁₂)-Cycloalkyl und (C₃-C₁₂)-Cycloalkyl-(C₁-C₆)-alkyl und Oxo, wobei der Rest Alkylen im Bereich der Kohlenstoff-Kohlenstoff-Bindung mit einem Carbocyclus oder einem Heterocyclus mit 5 bis 7 Ringgliedern und 1 oder 2 Stickstoffatomen, gesättigt oder ungesättigt und nicht substituiert oder substituiert durch R₃, verbunden sein kann;
R₃ eine Gruppe Alkyl oder Alkoxy mit 1 bis 6 Kohlenstoffatomen bedeutet;
R₄ ein Wasserstoffatom oder eine Gruppe Alkyl mit 1 bis 6 Kohlenstoffatomen darstellt,
R₅ ein Wasserstoffatom, eine Gruppe CO₂R₆, SO₂R₆, SO₂NHR₆ oder SO₂NHCO₂R₆ bedeutet, worin R₆ einen Rest (C₁-C₈)-Alkyl oder Naphthyl, nicht substituiert oder substituiert durch R₃, einen Rest Cycloalkyl mit 3 bis 12 Kohlenstoffatomen oder (C₃-C₁₂)-Oycloalkyl-(C₁-C₆)-alkyl oder den Rest der Formel (II) 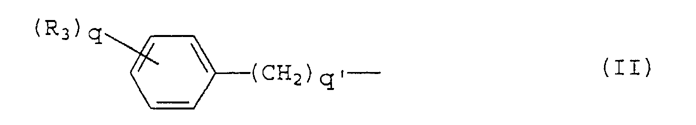 darstellt, worin die Reste R₃ gleich oder verschieden sein können und sich in irgendeiner Position des Restes Phenyl befinden können,
q und q' gleich 0 oder 1 sind;
m eine ganze Zahl von 2 ist;
n eine ganze Zahl von 1 ist;
wobei die genannten Reste Alkyl mono- oder polyungesättigt, die genannten Reste Cycloalkyl mono-, bi- oder tricyclisch, nicht substituiert oder substituiert durch ein oder mehrere (C₁-C₄)-Alkyl oder bei den Bi- und Tricycloalkylen durch ein oder mehrere Oxo, sein können, die genannten Reste Aryl oder Heteroaryl nicht substituiert oder substituiert durch ein oder mehrere gleiche oder verschiedene Reste, ausgewählt unter (C₁-C₈)-Alkyl, (C₁-C₈)-Alkoxy, Halogen, Nitro, Amino, Trifluormethyl, Hydroxyl, Methylendioxy, Cyano, Hydroxycarbonyl, Aminocarbonyl, (C₁-C₄)-Alkoxycarbonyl, Phenyl, Phenoxy, Benzyl und Benzyloxy, sein können,
wobei die genannten Verbindungen der Formel (I) in allen ihren möglichen stereoisomeren Formen, allein oder in Mischung in irgendeinem Verhältnis, vorliegen können, sowie ihre physiologisch akzeptablen Salze und ihre Prodrugs.

5. Verbindung der Formeln (I) oder (I') wie in irgendeinem der Ansprüche 1 bis 4 definiert, worin R₅ ein Rest CO₂R₆ ist und R₆ -CH₂Ph, -C(CH₃)₃ und CH₂-Adamantyl darstellt, wobei die genannten Verbindungen der Formel (I) in allen ihren möglichen stereoisomeren Formen, allein oder in Mischung in irgendeinem Verhältnis, vorliegen können, sowie ihre physiologisch akzeptablen Salze und ihre Prodrugs.

6. Verbindung der Formeln (I) oder (I') wie in irgendeinem der Ansprüche 1 bis 4 definiert, worin R₅ ein Rest SO₂R₆ ist und R₆ einen Rest Alkyl mit 1 bis 6 Kohlenstoffatomen, Naphthyl und Phenyl darstellt, substituiert durch einen oder mehrere Reste Alkyl mit 1 bis 6 Kohlenstoffatomen oder die Gruppe CF₃, wobei die genannter Verbindungen der Formel (I) in allen ihren möglichen stereoisomeren Formen, allein oder in Mischung in irgendeinem Verhältnis, vorliegen können, sowie ihre physiologisch akzeptablen Salze und ihre Prodrugs.

7. Verbindung der Formeln (I) oder (I') wie in irgendeinem der Ansprüche 1 bis 4 definiert, worin R₅ ein Rest SO₂NHR₆ oder SO₂NHCO₂R₆ ist und R₆ -CH₂Ph, -C(CH₃)₃ und CH₂-Adamantyl darstellt,
wobei die genannten Verbindungen der Formel (I) in allen ihren möglichen stereoisomeren Formen, allein oder in Mischung in irgendeinem Verhältnis, vorliegen können, sowie ihre physiologisch akzeptablen Salze und ihre Prodrugs.

8. Verbindung der Formeln (I) oder (I') wie in irgendeinem der Ansprüche 1 bis 7 definiert, mit den folgenden Namen:
- 5-{3-Oxo-3-[(1,4,5,6-tetrahydro-2-pyrimidinyl)-amino]-propyl}-.alpha.-{[(phenylmethoxy)-carbonyl]-amino}-2-benzofuran-propansäure,
- 5-{3-Oxo-3-[(1,4,5,6-tetrahydro-2-pyrimidinyl)-amino]-propyl}-.alpha.-{[(phenylmethoxy)-carbonyl]-amino}-2-benzofuran-propansäure-1-methylethylester,
- 5-{3-Oxo-3-[(1,4,5,6-tetrahydro-2-pyrimidinyl)-amino]-propyl}-.alpha.-{[(phenylmethoxy)-carbonyl]-amino}-2-benzofuran-propansäure-ethylester,
- 5-{3-Oxo-3-[(1,4,5,6-tetrahydro-2-pyrimidinyl)-amino]-propyl}-.alpha.-{[((tricyclo[3.3.1.1.^{3,7}]-dec-1-yl)methoxy)-carbonyl]-amino}-2-benzofuran-propansäure,
- 5-{3-Oxo-3-[(1,4,5,6-tetrahydro-2-pyrimidinyl)-amino]-propyl}-.alpha.-{{[[((tricyclo[2.3.1.1.^{3,7}]-dec-1-yl)methoxy)-carbonyl]-amino]-sulfonyl}-amino}-2-benzofuran-propansäure,
- 5-{3-Oxo-3-[(1,4,5,6-tetrahydro-2-pyrimidinyl)-amino]-propyl}-.alpha.-{[[((phenyl)-methoxy)-carbonyl]-amino]-sulfonyl}-2-benzofuran-propansäure,
- .alpha.-{[((Phenylmethyl)-amino)-sulfonyl]-amino}-5-{3-oxo-3-[(1,4,5,6-tetrahydro-2-pyrimidinyl)-amino]-propyl}-2-benzofuranpropansäure,
- .alpha.-{[(4-(1,1-Dimethylethyl)-phenyl)-sulfonyl]-amino}-5-{3-oxo-3-[(1,4,5,6-tetrahydro-2-pyrimidinyl)-amino]-propyl}-2-benzofuran-propansäure,
- .alpha.-{[(4-(1-Methylethyl)-phenyl)-sulfonyl]-amino}-5-{3-oxo-3-[(1,4,5,6-tetrahydro-2-pyrimidinyl)-amino]-propyl}-2-benzofuran-propansäure,
- 5-{3-Oxo-3-[(1,4,5,6-tetrahydro-2-pyrimidinyl)-amino]-propyl}-.alpha.-[(propyl-sulfonyl)-amino]-2-benzofuran-propansäure,
- .alpha.-[(Methyl-sulfonyl)-amino]-5-{3-oxo-3-[(1,4,5,6-tetrahydro-2-pyrimidinyl)-amino]-propyl}-2-benzofuran-propansäure,
- .alpha.-[(1-Naphthalin-sulfonyl)-amino]-5-{3-oxo-3-[(1,4,5,6-tetrahydro-2-pyrimidinyl)-amino]-propyl}-2-benzofuran-propansäure,
- .alpha.-[(1-Naphthalin-sulfonyl)-amino]-5-{3-oxo-3-[(1,4,5,6-tetrahydro-2-pyrimidinyl)-amino]-propyl}-2-benzofuran-propansäure-1-methylethylester,
sowie ihre physiologisch akzeptablen Salze und ihre Prodrugs.

9. Verfahren zur Herstellung der Verbindungen der Formel (I) wie in irgendeinem der Ansprüche 1 bis 8 definiert, das die Kupplung von zwei oder mehreren Fragmenten umfaßt, die durch Retrosynthese der Verbindungen der Formel (I) abgeleitet werden können.

10. Verfahren nach Anspruch 9, bei dem man die folgenden Stufen durchführt:
a) Bildung einer Carbonsäure oder eines Carbonsäure-Derivates der Formel (III) 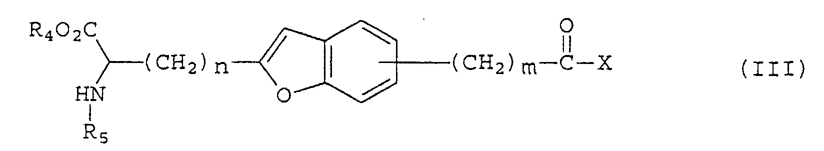 in der R₄, R₅, n und m wie in Anspruch 1 bei der Formel (I) definiert sind und X eine durch ein Nukleophil substituierbare Abgangsgruppe darstellt, durch Reaktion des Aminoesters der Formel (V) 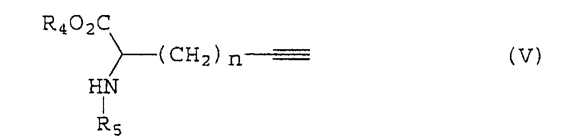 in der R₄, R₅ und n wie in Anspruch 1 bei der Formel (I) definiert sind, in Anwesenheit eines Katalysators und im basischen Medium mit einer Carbonsäure oder einem Carbonsäure-Derivat der Formel (VI) 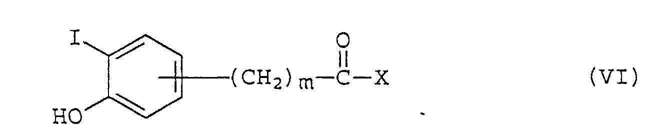 in der m und X wie weiter oben definiert sind, wobei die eventuellen funktionellen Gruppen, die in Form von Vorläufern oder in geschützter Form vorliegen, anschließend in Gruppen umgewandelt werden, die in den Verbindungen der Formel (I) anwesend sind, und
b) Kupplung der Carbonsäure oder eines Carbonsäure-Derivates der Formel (III) wie weiter oben definiert mit einem Guanidin oder einem Guanidin-Derivat der Formel (IV) 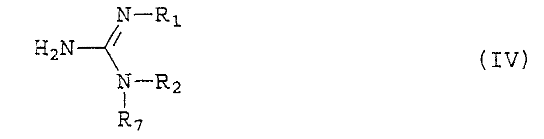 in der R₁, R₂ und R₇ wie in Anspruch 1 bei der Formel (I) definiert sind, wobei die eventuellen funktionellen Gruppen, die in Form von Vorläufern oder in geschützter Form vorliegen, anschließend in Gruppen umgewandelt werden, die in den Verbindungen der Formel (I) anwesend sind.

11. Als Arzneimittel eine Verbindung der Formel (I) und/oder ihre physiologisch akzeptablen Salze und/oder ihre Prodrugs wie in irgendeinem der Ansprüche 1 bis 8 definiert.

12. Pharmazeutische Zusammensetzung, umfassend mindestens eine Verbindung der Formel (I) wie in irgendeinem der Ansprüche 1 bis 8 definiert und/oder ihre physiologisch akzeptablen Salze und/oder ihre Prodrugs sowie einen oder mehrere übliche, inerte pharmazeutische Träger und gegebenenfalls einen oder mehrere Zusatzstoffe.

13. Als Arzneimittel mit einer antagonistischen Aktivität des Rezeptors von Vitronectin eine Verbindung der Formel (I) und/oder ihre physiologisch akzeptablen Salze und/oder ihre Prodrugs wie in irgendeinem der Ansprüche 1 bis 8 definiert.

14. Als Arzneimittel mit einer Inhibitor-Aktivität der Knochenresorption oder zur Behandlung oder Vorbeugung der Osteoporose eine Verbindung der Formel (I) und/oder ihre physiologisch akzeptablen Salze und/oder ihre Prodrugs wie in irgendeinem der Ansprüche 1 bis 8 definiert.

15. Als Arzneimittel mit einer Inhibitor-Aktivität für das tumorale Wachstum oder die kanzerogenen Metastasen eine Verbindung der Formel (I) und/oder ihre physiologisch akzeptablen Salze und/oder ihre Prodrugs wie in irgendeinem der Ansprüche 1 bis 8 definiert.

16. Als Arzneimittel mit einer anti-inflammatorischen Aktivität oder zur Behandlung oder Vorbeugung von cardiovasculären Störungen, Restenosen, Arteriosklerose, Nephropathien oder Retinopathien eine Verbindung der Formel (I) und/oder ihre physiologisch akzeptablen Salze und/oder ihre Prodrugs wie in irgendeinem der Ansprüche 1 bis 8 definiert.

17. Verwendung der Verbindungen der Formel (I) und/oder ihrer physiologisch akzeptablen Salze und/oder ihrer Prodrugs wie in irgendeinem der Ansprüche 1 bis 8 definiert, für die Herstellung von Arzneimitteln, die für die Vorbeugung oder die Behandlung der Osteoporose vorgesehen sind.

18. Verwendung der Verbindungen der Formel (I) und/oder ihrer physiologisch akzeptablen Salze und/oder ihrer Prodrugs wie in irgendeinem der Ansprüche 1 bis 8 definiert, für die Herstellung von Arzneimitteln, die für die Inhibierung des tumoralen Wachstums oder der kanzerogenen Metastasen vorgesehen sind.

19. Verwendung der Verbindungen der Formel (I) und/oder ihrer physiologisch akzeptablen Salze und/oder ihrer Prodrugs wie in irgendeinem der Ansprüche 1 bis 8 definiert, für die Herstellung von Arzneimitteln, die für die Vorbeugung oder Behandlung von cardiovasculären Störungen, Restenosen, Arteriosklerose, Nephropathien oder Retinopathien vorgesehen sind.
